(19) Europäisches Patentamt / European Patent Office / Office européen des brevets

(11) **EP 3 887 535 B1**

(12) **EUROPEAN PATENT SPECIFICATION**

(45) Date of publication and mention
of the grant of the patent:
**15.05.2024 Bulletin 2024/20**

(21) Application number: **19816394.1**

(22) Date of filing: **26.11.2019**

(51) International Patent Classification (IPC):
***C12Q 1/18*** *(2006.01)*

(52) Cooperative Patent Classification (CPC):
**C12Q 1/18**

(86) International application number:
**PCT/GB2019/053324**

(87) International publication number:
**WO 2020/109764 (04.06.2020 Gazette 2020/23)**

(54) **ANTIMICROBIAL SUSCEPTIBILITY ASSAY AND KIT**

ASSAY UND KIT ZUR BESTIMMUNG ANTIMIKROBIELLER SUSZEPTIBILITÄT

ESSAI ET TROUSSE POUR L'ÉVALUATION DE LA SUSCEPTIBILITÉ ANTIMICROBIENNE

(84) Designated Contracting States:
**AL AT BE BG CH CY CZ DE DK EE ES FI FR GR HR HU IE IS IT LI LT LU LV MC MK MT NL NO PL PT RO RS SE SI SK SM TR**

(30) Priority: **26.11.2018 GB 201819187**

(43) Date of publication of application:
**06.10.2021 Bulletin 2021/40**

(73) Proprietor: **The University of Liverpool
Liverpool, L69 3GL (GB)**

(72) Inventors:
• **KELL, Douglas
Manchester M13 9PL (GB)**
• **JINDAL, Srijan
Manchester M13 9PL (GB)**

(74) Representative: **Appleyard Lees IP LLP
15 Clare Road
Halifax HX1 2HY (GB)**

(56) References cited:
**WO-A1-2018/184073      WO-A2-2010/129532
US-A1- 2003 103 936      US-A1- 2018 195 964**

• Jose V Ordonez ET AL: "Rapid Flow Cytometric Antibiotic Susce tibility Assay for Staphylococcus aureus P", Cytometry, 1 January 1993 (1993-01-01), pages 811-818, XP055320404, Retrieved from the Internet: URL:http://onlinelibrary.wiley.com/store/10.1002/cyto.990140714/asset/990140714_ftp.pdf?v=1&t=ivm755mw&s=13ee776b201dcf394031a 3eb304c12796d29a6e3
• MASON D J ET AL: "The ability of membrane potential dyes and calcafluor white to distinguish between viable and non-viable bacteria", JOURNAL OF APPLIED BACTERIOLOGY, BLACKWELL PUBLISHING LTD., OXFORD, GB, vol. 78, no. 3, 1 March 1995 (1995-03-01), pages 309-315, XP009188807, ISSN: 0021-8847
• Valerie S Forsyth ET AL: "Rapid Growth of Uropathogenic Escherichia coli during Human Urinary Tract Infection", mBio, 6 March 2018 (2018-03-06), pages e00186-18, XP055664343, United States DOI: 10.1128/mBio.00186-18 Retrieved from the Internet: URL:https://mbio.asm.org/content/mbio/9/2/e00186-18.full.pdf
• YANG S T ET AL: "Different modes in antibiotic action of tritrpticin analogs, cathelicidin-derived Trp-rich and Pro/Arg-rich peptides", BIOCHIMICA ET BIOPHYSICA ACTA (BBA) - BIOMEMBRANES, ELSEVIER, AMSTERDAM, NL, vol. 1758, no. 10, 1 October 2006 (2006-10-01), pages 1580-1586, XP005681671, ISSN: 0005-2736 [retrieved on 2006-10-01]

EP 3 887 535 B1

- **SRIJAN JINDAL ET AL: "Very rapid flow cytometric assessment of antimicrobial susceptibility during the apparent lag phase of microbial (re)growth", JOURNAL OF GENERAL MICROBIOLOGY, vol. 165, no. 4, 1 April 2019 (2019-04-01) , pages 439-454, XP055664172, ISSN: 1350-0872, DOI: 10.1099/mic.0.000777**

Remarks:

The file contains technical information submitted after the application was filed and not included in this specification

**Description**

**Technical Field of the Invention**

This invention relates to rapid assays for detecting antimicrobial susceptibility in samples from patients suffering from a microbial infection, as defined in the claims. The invention is particularly suited for detecting antimicrobial susceptibility in urine from patients suffering from a Urinary Tract Infection ("UTI").

**Background to the Invention**

As is well known, there is a crisis of resistance to antimicrobials (Andersson and Hughes 2010; Baker et al. 2018; Gelband and Laxminarayan 2015; Laxminarayan et al. 2016; Li et al. 2016; Macedo et al. 2013; Mendelson et al. 2017; Roca et al. 2015), caused in part by mis-prescribing. This mis-prescribing takes two forms: (i) potentially effective antibiotics are given when the infection is not bacterial, or (ii) the wrong (i.e. ineffective) antibiotics are given when it is. What would be desirable would be a very rapid means of knowing, even before a patient left a doctor's surgery, that a particular antibiotic was indeed capable of killing the organism of interest. While genotypic (whole-genome-sequencing) methods hold out some promise for this (Buchan and Ledeboer 2014; Didelot et al. 2012; Dunne et al. 2017; Kirkup et al. 2013; Köser et al. 2012; Kwong et al. 2015; Roach et al. 2015; Schmidt et al. 2016; Tsai et al. 2016; Tuite et al. 2014; van Belkum and Dunne 2013), what is really desired is a phenotypic assay (Farha and Brown 2010) that assesses the activity of anti-infectives in the sample itself (Kelley 2017; Murray et al. 2015; Schmiemann et al. 2010). However, since almost all antibiotics, whether bacteriostatic or bactericidal (Kohanski et al. 2007), indicate their efficacy or otherwise only when cells are attempting to replicate (Coates et al. 2011), it might be thought that this would be an unattainable goal simply because of the existence of a lag phase (described below).

Urinary tract infections ("UTIs") are a worldwide patient problem (Schmiemann et al. 2010). Other than in hospital-acquired infections (Cek et al. 2014), they are particularly common in females, with 1 in 2 women experiencing a UTI at some point in their life (Foxman 2010). *Escherichia coli* is the most common causative pathogen of a UTI (Ejrnaes 2011; Foxman 2010; Mehnert-Kay 2005; Wilson and Gaido 2004). However, other Enterobacteriaceae such as *Proteus mirabilis, Klebsiella* spp. and *Pseudomonas aeruginosa*, and even Gram-positive cocci such as staphylococci and enterococci, may also be found (Kline and Lewis 2016; Tandogdu and Wagenlehner 2016). Misapplication and overuse of antibiotics in primary care is a major source of antimicrobial resistance (Bryce et al. 2016; Cek et al. 2014; Tandogdu and Wagenlehner 2016), so it is important that the correct antibiotic is prescribed (Kerremans et al. 2008; Kirchhoff et al. 2018). Often prescribing none at all for asymptomatic UTIs is an adequate strategy (Köves et al. 2017).

We note, however, that *E. coli cells* in all conditions are highly heterogeneous (Kell et al. 2015), even if only because they are in different phases of the cell cycle (Wallden et al. 2016), and in both 'exponential' and stationary phase contain a variety of chromosome numbers (Åkerlund et al. 1995; Boye and Lobner-Olesen 1991; Skarstad et al. 1986; Skarstad et al. 1985; Steen and Boye 1980; Stokke et al. 2012). To discriminate them physiologically, and especially to relate them to culturability (a property of an individual), it is necessary to study them individually (Kell et al. 1991; Taheri-Araghi et al. 2015), typically using flow cytometry (Boye et al. 1983; Davey 2011; Davey and Kell 1996; Hewitt and Nebe-Von-Caron 2004; Kell et al. 1991; Müller et al. 1993; Nebe-von Caron and Badley 1995; Shapiro 2008, 2001; Shapiro 2003; Steen 1990). Flow cytometry has also been used to count microbes (and indeed white blood cells) for the purposes of assessing UTIs (Chien et al. 2007; Koken et al. 2002; Shang et al. 2013; Shayanfar et al. 2007; Wang et al. 2010), but not in these cases for antibiotic susceptibility testing. Single cell morphological imaging has also been used, where in favourable cases antibiotic susceptibility can be detected in 15-30 minutes or less (Baltekin et al. 2017; Choi et al. 2014).

A number of workers have recognised that flow cytometry has the potential to detect very rapid changes in both cell numbers, morphology (by light scattering) and physiology (via the addition of particular fluorescent stains that report on some element(s) of biochemistry or physiology). Boye and colleagues could see effects of penicillin on flow cytograms within an hour of its addition to sensitive strains (Boye et al. 1983). Similarly, Gant and colleagues (Gant et al. 1993) used forward and side scattering, and noticed antibiotic-dependent effects on the profiles after 3h, but did not measure absolute counts. Later studies (Mason et al. 1994; Mason et al. 1995) used the negatively charged dye bis-(1,3-dibutyl-barbituric acid) trimethine oxonol (DiBAC4(3)), which increases its binding and hence fluorescence upon loss of membrane energisation (that decreases the activity of efflux pumps such as acrAB/tolC (Du et al. 2018; Iyer et al. 2015; Kessel et al. 1991)), and could detect susceptibility to penicillin and gentamicin in 2-5h. Using a similar assay, Senyurek and colleagues (Senyurek et al. 2009) could detect it within 90 min. Other workers have used a variety of probes, but evaluation was after a much longer period, e.g. 24h (Boi et al. 2015). Álvarez-Barrientos and colleagues (Álvarez-Barrientos et al. 2000) give an excellent review of work up to 2000, with some reports (e.g. (Walberg et al. 1997)) of detection of flow cytometrically observable changes in morphology (light scattering) at 30 min exposure to antibiotic. Flow cytometry has also been used to detect bacteriuria, although the numbers found seemed not to correlate well with CFUs (Pieretti et al. 2010). Most so-called 'live/dead' kits rely on the loss of membrane integrity to detect the permeability of DNA stains, but many effective antibiotics have little effect on this in the short term, and such kits do not assess proliferation (Kell et al. 1998).

A classical activity of general and laboratory microbiology involves the inoculation of a liquid nutrient broth with cells taken from a non-growing state, whether this be from long-term storage (typically in agar) or using cells that have been grown to stationary phase (Navarro Llorens et al. 2010), more or less recently, in another liquid batch culture. The result of this is that the cells will, in time, typically increase in number and/or biomass, often exponentially, but that this is preceded by a 'lag phase' (that may be subdivided (Schultz and Kishony 2013)) before any such increases. The length of the lag phase depends on various factors, including the nature of the nutrient media before and after inoculation, the inoculum density, pH, temperature, and the period of the previous stationary phase for that cell (Bertrand 2014; Finkel 2006; Himeoka and Kaneko 2017; Jõers and Tenson 2016; Pin et al. 2009; Roostalu et al. 2008; Swinnen et al. 2004). It is usually estimated (and indeed defined) by extrapolating to its starting ordinate value a line on a plot of the logarithm of cell number, cfu or biomass against time (e.g. (Baranyi and Pin 1999; Baty and Delignette-Muller 2004; Baty et al. 2002; Pirt 1975; Prats et al. 2008; Prats et al. 2006; Swinnen et al. 2004)). However, because of the different (and generally lower) sensitivity of bulk optical estimates of biomass (Dalgaard et al. 1994; Madar et al. 2013; Swinnen et al. 2004), only the first two of these are normally considered to estimate the 'true' lag phase.

With some important exceptions (e.g. (Link et al. 2015; Madar et al. 2013; Novotna et al. 2003; Pin et al. 2009; Rolfe et al. 2012; Roostalu et al. 2008), the lag phase has been relatively little studied at a molecular level. From an applied point of view, however, at least two influences on it are considered desirable. Thus, a food microbiologist might wish to maximise the lag phase (potentially indefinitely) (e.g. (Baranyi and Roberts 1994)). By contrast, there are circumstances, as here, and not least in clinical microbiology, where it is desirable to be able to measure microbial growth/culturability, and its phenotypic sensitivity or otherwise to candidate anti-infective agents, in as short a time as possible. This necessarily involves minimising the length of the lag phase, and is the focus of the present studies.

There is evidence that the time before measurable biochemical changes occur during lag phases can be very small when inoculation is into rich medium (Link et al. 2015; Madar et al. 2013; Rolfe et al. 2012). Thus, Rolfe and colleagues (Rolfe et al. 2012) used Lysogeny Broth (LB) (and *S. enterica*), where lag phase or regrowth - as measured by changes in the transcriptome - initiated within 4 min (the earliest time point measured). The timescale in the plots of Madar and colleagues (Madar et al. 2013) does not admit quite such precise deconvolution, but responses in M9 with casamino acids (referred to as 'immediate') are consistent with a period of less than 10 min. Hong and colleagues recently detected such changes in under 30 min using stimulated Raman imaging (Hong et al. 2018), Yu and colleagues could do so with video microscopy (Yu et al. 2018), and Schoepp *et al.* (Schoepp et al. 2017) used molecular detection of suitable transcripts.

WO2018/184073 discloses methods for the qualitative and quantitative determination of the susceptibility of microbial organisms such as bacteria to antimicrobial agents such as antibiotics.

WO2010/129532 discloses a method and a device for expediting delivery of an agent to a damaged bacterial cell.

US2018/195964 discloses single dye fluorescent staining and the combination of differences in both intensity and spectral emission permit determination of the minimum concentration of an antibiotic needed to inactivate bacteria (Minimum Inhibitory Concentration (MIC)), thereby providing a means for rapid antibiotic susceptibility testing.

J V Ordonez et al, (1993) Cytometry, 14, (7), pages 811 - 818, discloses a rapid flow cytometric antibiotic susceptibility assay for Staphylococcus aureus.

Mason A D J et al, (1995) J. Appl. Bacteriol. vol. 78, no. 3, pages 309 - 315, discloses the ability of membrane potential dyes and calcofluor white to distinguish between viable and non-viable bacteria.

V S Forsyth et al, (2018), American Societ. Biol., 9, (2), e00186-181 - 18, pages 1 - 8, discloses rapid growth of uropathogenic *Escherichia coli* during human urinary tract infection.

YANG S T et al, (2006) Biochemica et Biophysica Acta, 1758, (10), pages 1580 - 1586, disclose different modes in antibiotic action of tritrpticin analogs, cathelicidin-derived Trp-rich and Pro/Arg-rich peptides.

US2003/103936 discloses ex-vivo expansion of hematopoietic cells by culturing hematopoietic cells in a growth medium.

An object of the present invention is to provide an assay for detecting antimicrobial susceptibility in urine samples from patients suffering from a microbial infection. It is desirable that the assay be rapid and easy to conduct so as to be able to provide a fast and accurate result which would enable a physician to prescribe an antimicrobial therapy that would successfully treat the microbial infection. Ideally, the assay should be able to be used on a range of bodily fluids, such as blood, urine, mucus or saliva. In the invention, however, the method is limited to urine samples. It would also be desirable to provide a assay for detecting antimicrobial susceptibility in urine from patients suffering from UTIs.

## Summary of the Invention

The present invention is defined with reference to the appended claims.

## Summary of the Disclosure

There is disclosed, a method for rapidly determining the susceptibility of a microorganism to an antimicrobial agent comprising the steps:

a) contacting a first sample containing the microorganism with a first growth medium so as to form a first mixture, wherein the first growth medium is selected to enable the microorganism to proliferate and/or encourage the microorganism cell cycle to commence proliferation;

b) contacting a second sample containing the microorganism with a second growth medium so as to form a second mixture, wherein the second growth medium is substantially the same as the first growth medium but further comprises a first antimicrobial agent which may inhibit or slow the proliferation of the microorganism;

c) incubating the first and second mixtures, for 30 minutes or less, under conditions suitable to enable or encourage proliferation of the microorganism;

d) passing the first and second mixture, or portion thereof, through a flow cytometer in order to assess one or more biochemical and/or biophysical parameters of the microorganisms in both mixtures; and

e) comparing the parameters of the microorganisms in the first mixture with that of the second mixture, after incubation, in order to detect whether the first antimicrobial agent inhibits or slows the proliferation of the microorganism so as to determine the susceptibility of a microorganism to said agent.

In the method, step b) may further comprise contacting one or more further samples containing the microorganism with a one or more further growth media so as to form one or more further mixtures, wherein the one or more further growth media is the same as the first growth medium but further comprises one or more further antimicrobial agents which may inhibit or slow the proliferation of the microorganism, wherein said one or more further antimicrobial agents are different from one another and different from the first antimicrobial agent. Therefore, the provision of assessing one or more further samples with one or more further antimicrobial agents allows the method to assess the susceptibility of the microorganism to multiple antimicrobial agents. In a clinical setting, this would enable the physician to quickly identify which antimicrobial agent would be most successful in treating an infection.

In the method, step d) may additionally be conducted prior to and after step c) so that the one or more biochemical and/or biophysical parameters of a sample can be assessed prior to incubation.

The one or more biochemical and/or biophysical parameters of the microorganisms may be selected from one or more of the following: cell size, cell number, cell membrane energisation and/or nucleic acid content and/or distribution. The one or more biochemical and/or biophysical parameters of the microorganisms may be determined by assessing the uptake of one or more certain fluorescent or other stains.

The method has a number of advantages. Firstly, the method can identify susceptibility of the microorganism to antimicrobial agents in a time frame of 30 minutes or less. This rapid method would allow a physician or medical professional (such as a nurse) to take a sample from a patient suspected or known to have an infection and then identify the most suitable and most effective antimicrobial agent in order to treat the infection.

When the parameter is cell size, cell number and/or cell membrane energisation, preferably, the medium further comprises a carbocyanine dye, or prior to step d), carbocyanine dye is added to the mixture or part of the mixture. A preferred carbocyanine dye comprises 3,3'-dipropylthiadicarbocyanine iodide (di-S-C3(5)). The carbocyanine dye may be added to the growth medium or mixture so that it is present at a concentrate in the range of about 1 $\mu$M to about 5 $\mu$M, and preferably in the range of about 3 $\mu$M. When the parameter is cell size, cell number and/or cell membrane energisation, preferably, the flow cytometer relies upon excitation at a particular wavelength and assessment in a particular wavelength range. For example, the the flow cytometer may rely upon excitation at 640 nm and the parameters are assessed at 675±15 nm. Alternative wavelengths will also be evident to the skilled addressee. For example, the flow cytometer may rely upon excitation at around 633 or 638 nm.

When the parameter is nucleic acid, preferably, said nucleic acid comprises DNA. Prior to step d), mithramycin and/or a nucleic acid stain may be added to the mixture or part of the mixture, and optionally, DNA distribution is assessed on the flow cytometer at around 572 nm. When the parameter is DNA analyses, preferably, the cells are fixed by injection into ethanol, washed twice by centrifugation in a M-Tris/HCl buffer, before resuspension in the same buffer containing mithramycin and/or nucleic acid stain, $MgCl_2$, and NaCl. The nucleic acid stain may comprise a cyanine, such as SYBR Green or ethidium bromide. More preferably, cells are fixed by injection into ice-cold ethanol to a final concentration of about 70%, washed twice by centrifugation in 0.1 M-Tris/HCl buffer, about pH 7.4, before resuspension in the same buffer containing mithramycin (50 $\mu$g mL-1) and ethidium bromide (25 $\mu$g mL-1), $MgCl_2$, (25 mM) and NaCl (100 mM).

It will be apparent that the growth medium employed would be determined by the type of microorganism for which the sample is being tested and/or from which type of environment the microorganism has been removed. For example, the growth medium of Terrific Broth has been shown to be effective as a growth medium when assessing a microorganism in urine samples from individuals suffering from a urinary tract infection.

Likewise, step c) will take place at a temperature which is suitable for the proliferation of the microorganism for which the sample is being tested and/or from which type of environment the microorganism has been removed. For example, step c) will take place at a temperature in the range of about 35°C and 40°C, and preferably at a temperature of about 37°C, when assessing a microorganism in samples from individuals suffering from an infection.

A portion of the first and second mixture, or portion of the one or more further mixtures may be assessed at multiple time points. It is preferred that the multiple time points comprise one or more of the following time points, 0 minutes, 5 minutes, 10 minutes, 15 minutes, 20 minutes, 25 minutes and/or 30 minutes or any additional or alternative intervening time points or selection of time points thereof. Preferably, step c) is about 20 minutes or less. Most preferably, step c) is in the range of about 15 minutes to about 20 minutes.

Ideally, a portion of the first and second mixture, or portion of the one or more further mixtures is assessed prior to, and after, step c). This allows for a reference of the microorganism in a growth arrested state verses the microorganism in a state of proliferate and/or a state of cell cycle change in order to commence proliferation.

It will apparent that the microorganism may be obtained from a biological sample derived from an individual believed to be suffering from a microorganism infection. The microorganism may be obtained from a biological sample derived from an individual believed to be suffering from a bacterial or viral infection in which case the method may be used to assess whether the infection is of a bacterial or viral nature and help to direct the therapeutic or course of treatment. Preferably, the method will identify a change in one or more biochemical and/or biophysical parameters of the microorganisms against one or more antibacterial agents so as determine that the patient is suffering from a bacterial infection where the bacteira is susceptible for the antibactieral agent. However, if method does not identify a change in one or more biochemical and/or biophysical parameters of the microorganisms against any of the antibacterial agents then this may be determantive that the patient is suffering from a viral infection and the appropriate course of action (such as the administration of antivirals) taken.

The samples may be derived directly from body fluids, such as urine, blood, mucus or saliva. Alternatively, the samples may be pre-treated with reagents or buffers or filtered prior to being contacted with the growth medium.

The microorganism infection may be a Urinary Tract Infection (UTI).

There may be provided a method as herein above described for determining the antimicrobial agent for use in the treatment of a microorganism infection in an individual, wherein the method comprises taking a biological sample from the individual, assessing the susceptibility of the microorganism, in the biological sample, to two or more antimicrobial agents and identifying which antimicrobial agent to administer to the individual based which antimicrobial agent inhibits or slows the proliferation of the microorganism.

There may also be provided a kit for rapidly determining the susceptibility of a microorganism to an antimicrobial agent comprising:

a) an enriched growth medium;

b) one or more antimicrobial agents; and

c) a carbocyanine dye.

The kit may further comprise:
d) a flow cytometer, optionally, with at leaset one red laser.

It is preferable that the enriched growth medium comprises Terrific Broth, although it will be apparent that other enriched growth media may also be used and may be directed by the microorganism suspected to be in a sample.

It will be understood that the kit as herein above described, may be for use in the method as hereinabove described.

Unexpectedly and advantageously, the inventors had recognising that bacteria in UTIs may actually be growing (albeit slowly) and not in a 'stationary' phase, so they decided to assess the ability of quantitative flow cytometry to determine bacterial cell numbers, and the effects of antibiotics thereon, on as rapid a timescale as possible. Their findings show that it is indeed possible to discriminate antibiotic-susceptible and -resistant strains in under 30 mins at levels ($10^{4-5}$.mL$^{-1}$) characteristic (Detweiler et al. 2015; Mody and Juthani-Mehta 2014) of bacteriuria. Advantageously, this opens up the possibility of ensuring that a correct prescription is given to the patient at the surgery due to the short timing between a sample being taken and the identification of the correct antibiotic which can be prescribed and/or dispensed whilst the patient is still at the surgery.

Because different probes and different antibiotics have different effects (and with different kinetics) on membrane integrity, it was decided that the best strategy would be to look at the ability of antibiotics to inhibit proliferation directly, distinguishing bacteria from non-living scattering material via the use of a positively charged dye that energised living cells accumulate. Rhodamine 123 is a very popular dye of this type, but without extra chemical treatments that would inhibit proliferation is effective only in Gram-positive organisms (Kaprelyants and Kell 1993, 1992). However, the positively charged carbocyanine dye 3,3'-dipropylthiadicarbocyanine iodide (di-S-C3(5)) (Waggoner 1976; Waggoner 1979) seems to bind to and/or be accumulated by both Gram-positive and -negative bacteria, and provides a convenient means of detecting them.

## Detailed Description of the Invention

The invention is described below, by way of example only, with reference to the accompanying figures in which:

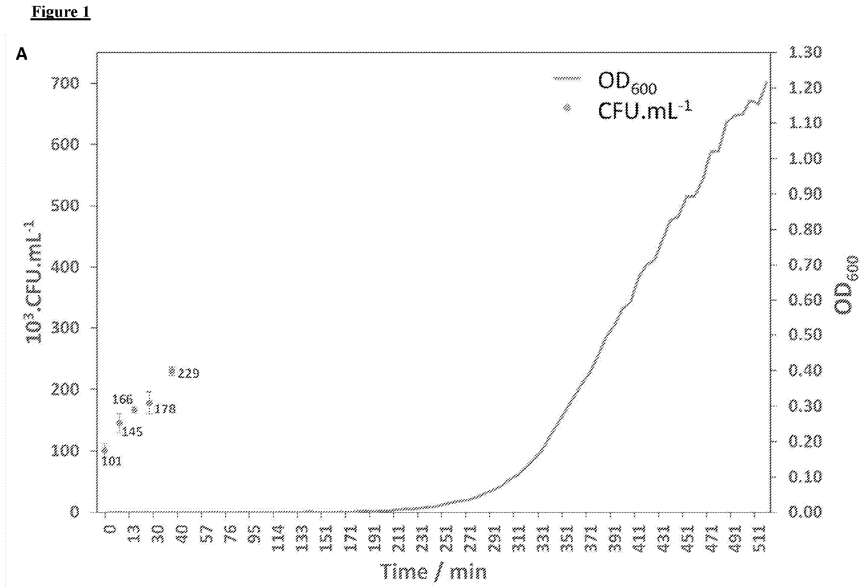

Figure 1. True and apparent lag phases during microbial regrowth. The strains indicated were grown in Lysogeny Broth and inoculated into Terrific Broth after 4 h in stationary phase to ca $10^5$ cells.mL$^{-1}$. OD was measured quasi-continuously in an Omega plate reader spectrophotometer (BMG Labtech, UK), while CFU were measured conventionally on agar plates containing nutrient agar medium solidified with 1.5% agar. The lag phase observed via counting CFUs is less than 30 min while bulk OD measurements show a lag phase of some 230 minutes (~4 hours).

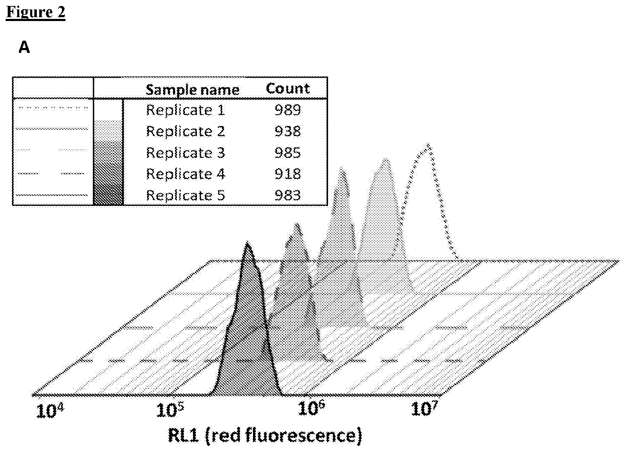

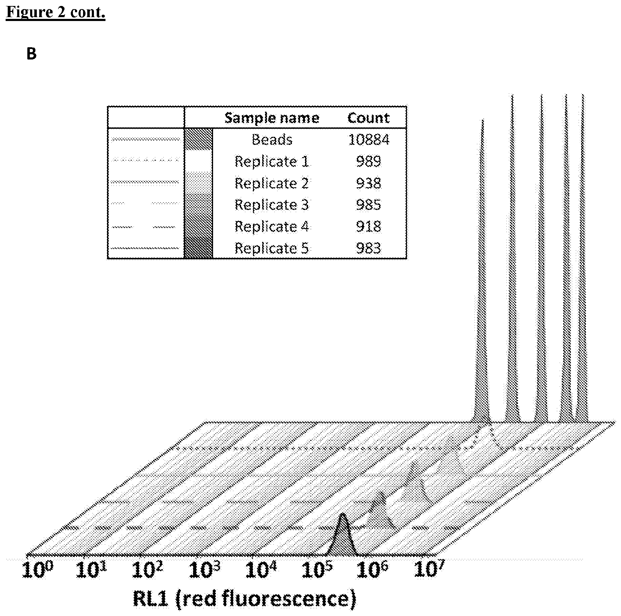

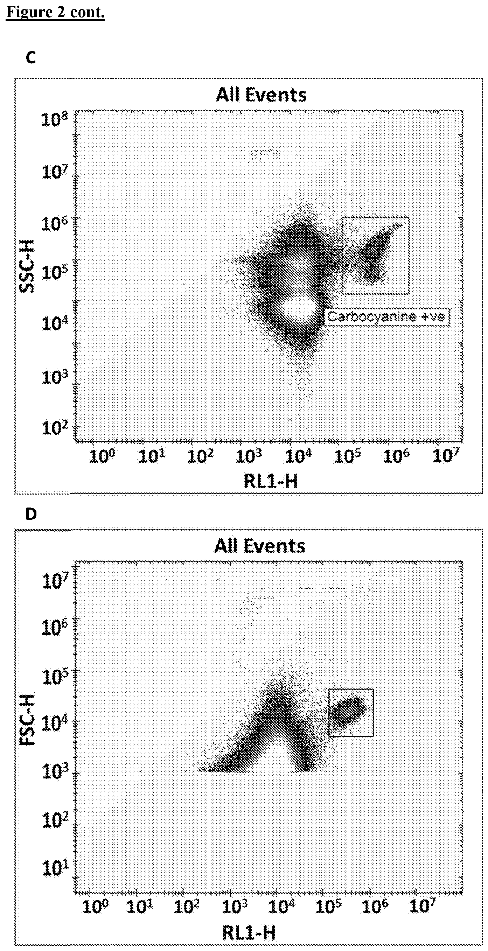

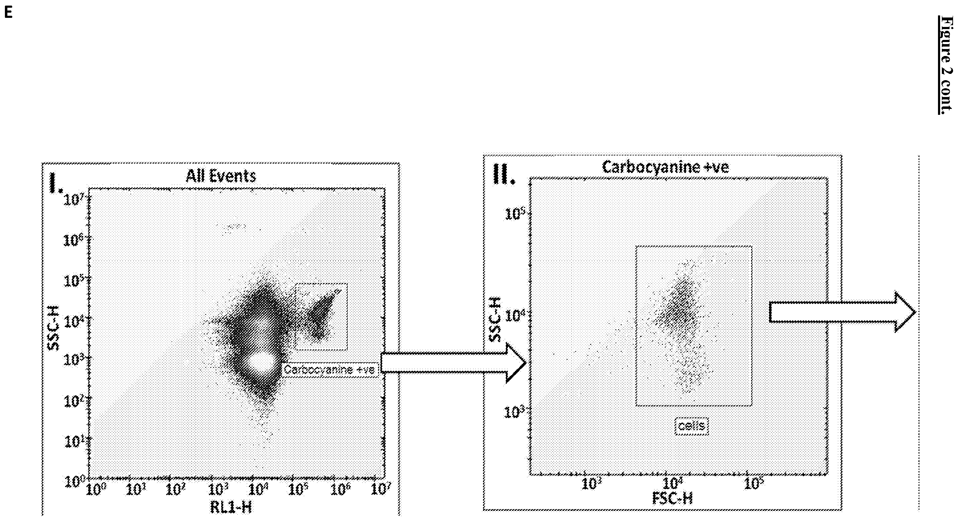

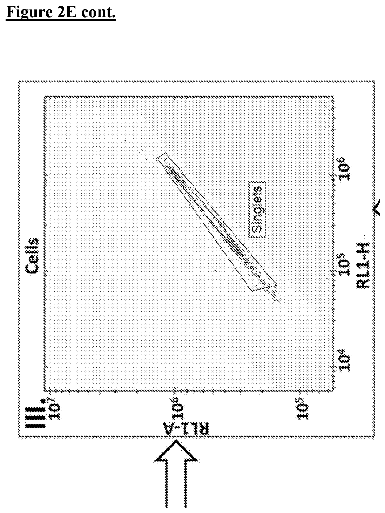

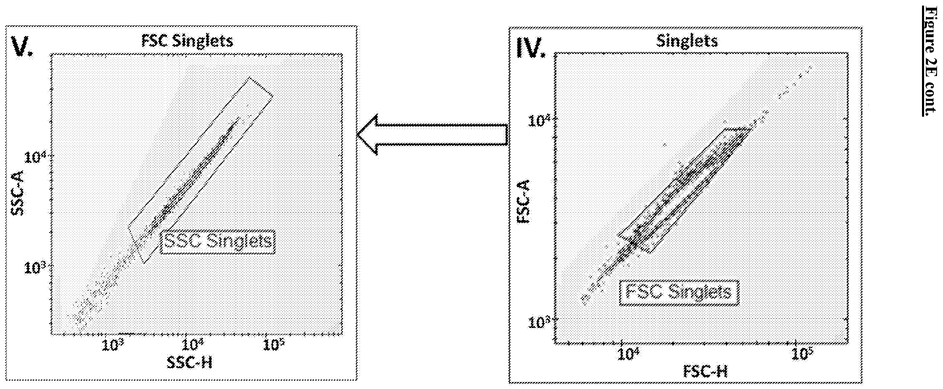

Figure 2. Cytograms of *E. coli* at a concentration of $10^5$ cells.mL$^{-1}$ when incubated in 0.2 μm-filtered Terrific Broth containing 3μM Di-S-C3(5). The sample measured has a volume of 3μL and measurement takes place over 2 seconds. **A**. 1D histograms of RL1 fluorescence showing the reproducibility of the results. **B**. 1D histograms of RL1 fluorescence together with calibrating beads, showing that the breadth of the bacterial peaks is 'real' and not simply due to detector variability. **C** and **D**. Raw dot plots of the height of the forward scatter and of side scatter signals respectively vs RL1. Note that the *E. coli* cells appear above a value of $10^5$ in RL1, the rest of the signals being due to very tiny unfiltered debris. **E**. Gating strategy (I-V) to show only the *E. coli* singlet cells.

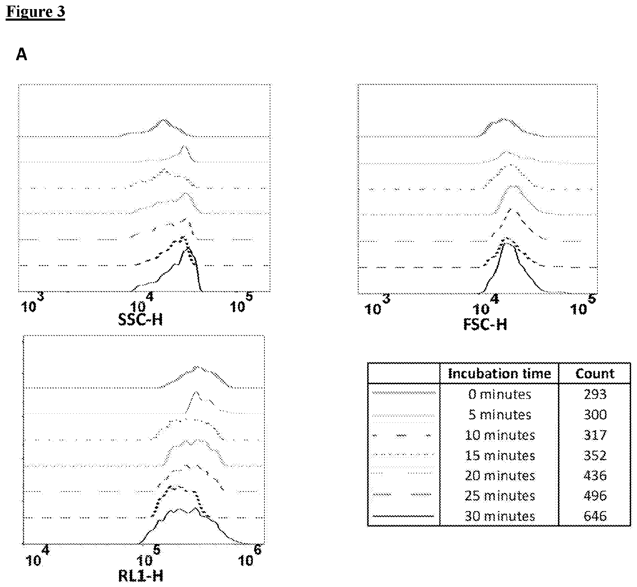

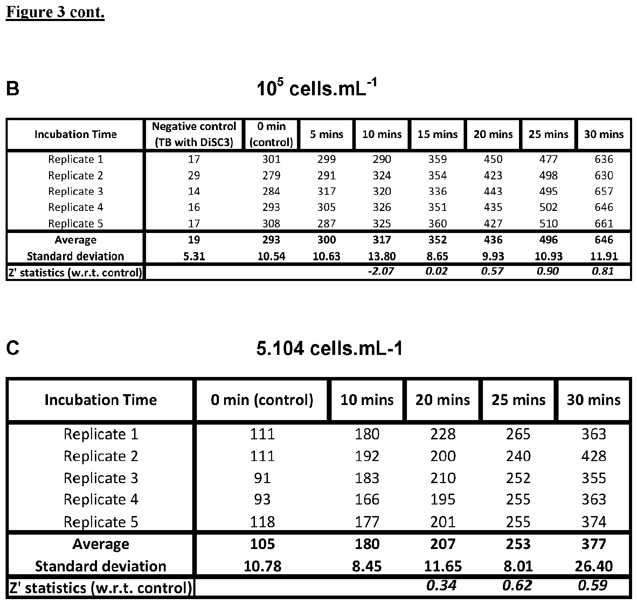

Figure 3. Changes in cell number during first 30 min following inoculation of cells from stationary phase into Terrific Broth. **A**. Typical cytograms. **B**. Reproducibility and Z' statistics (see below) for *E. coli* growth at initial concentration of $10^5$ cells.mL$^{-1}$. **C**. Reproducibility and Z' statistics for *E. coli* inoculated at $5\times10^5$ cells.mL$^{-1}$.

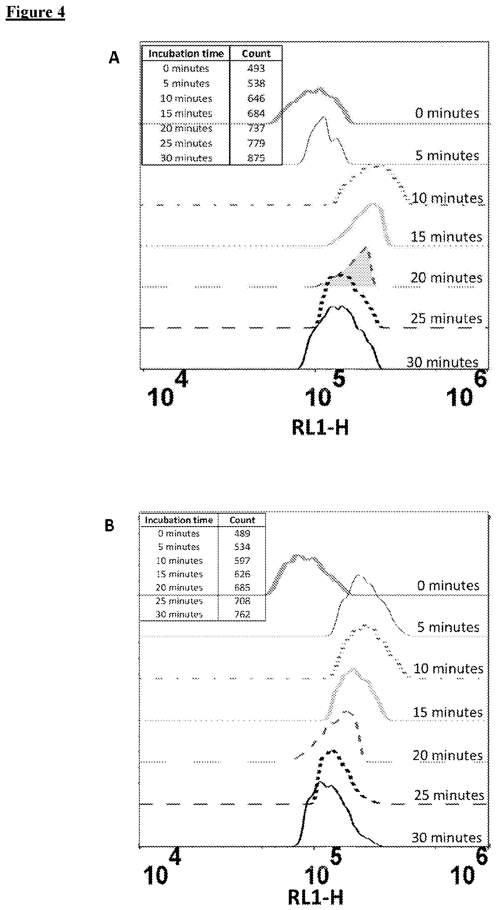

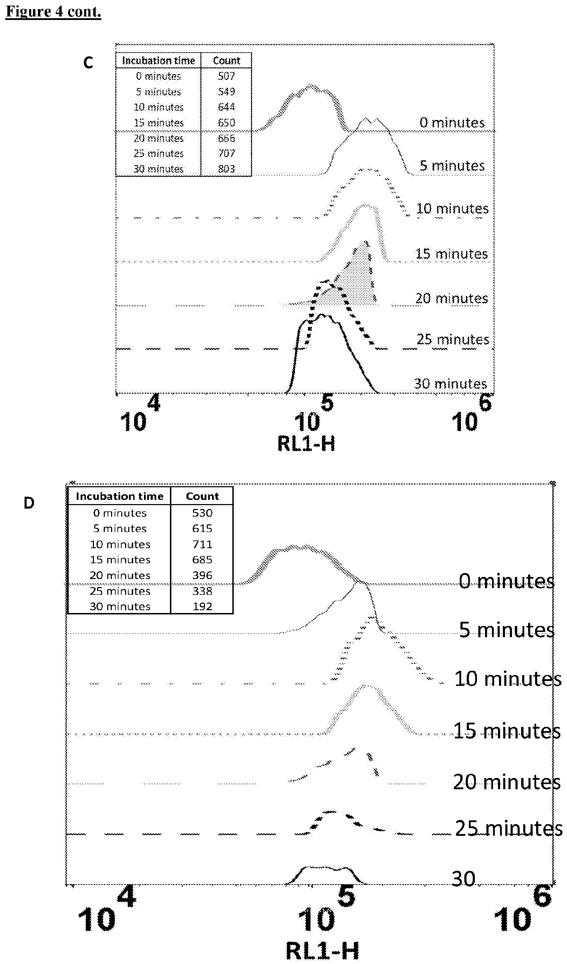

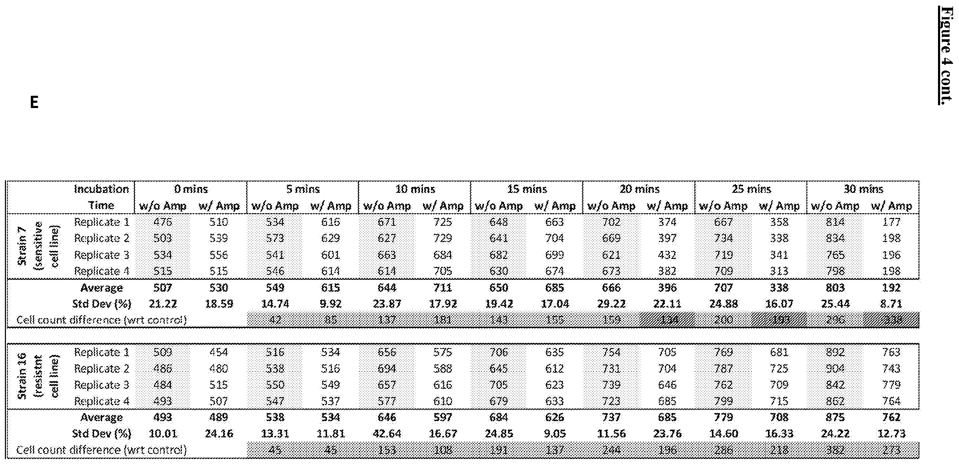

Figure 4. Effect of ampicillin (100 mg.L$^{-1}$ concentration, $3 \times$ MIC for sensitive strains) on the cytograms of *E. coli* inoculated from stationary phase into Terrific Broth. Ampillicin was either absent (**A,C**) or present (**B,D**) from resistant strain 16 (**A,B**) or sensitive strain 7 (**C,D**). **E**. Table showing the changes in the number of bacteria (with replicates) from sensitive (strain 7) and resistant strains (strain 16) when grown in the presence and absence of Ampicillin. Similar data were obtained using eight other macroscopically sensitive and resistant strains.

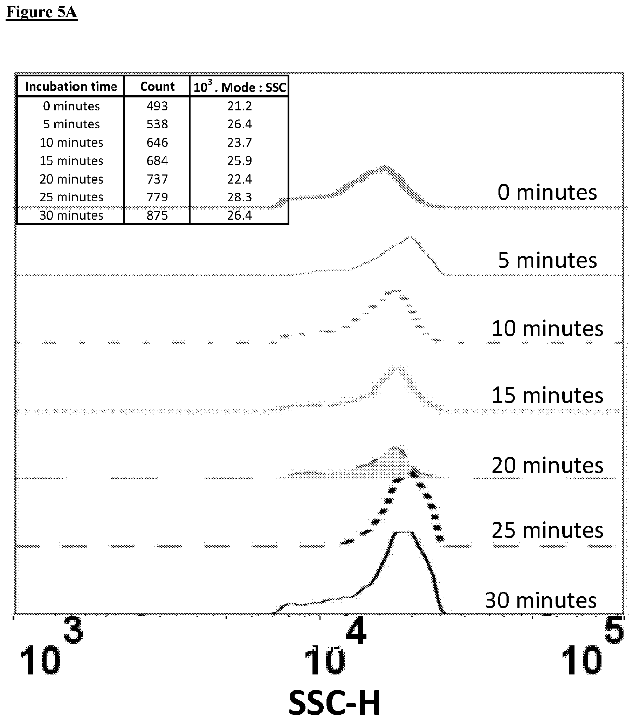

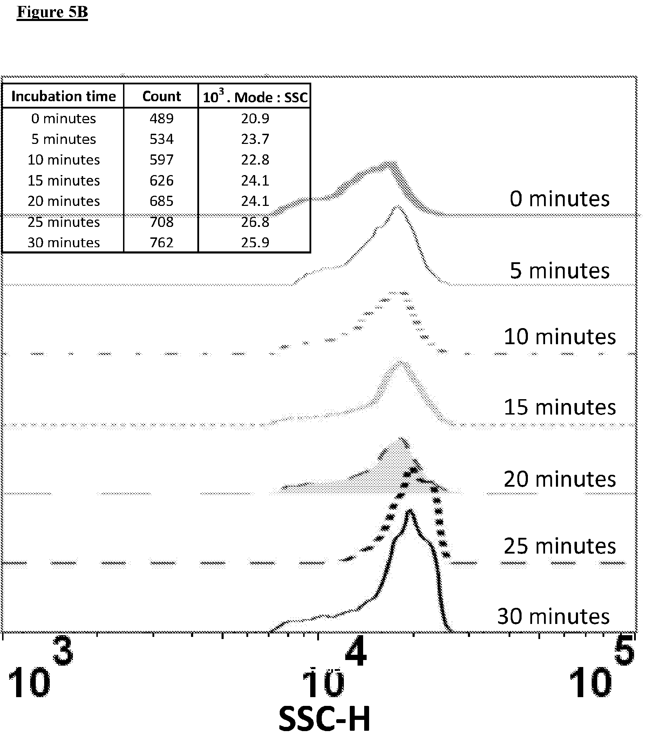

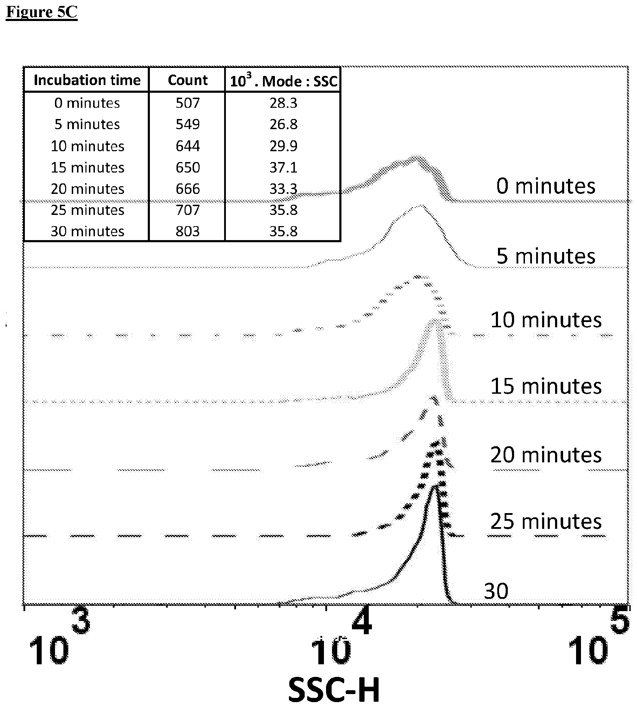

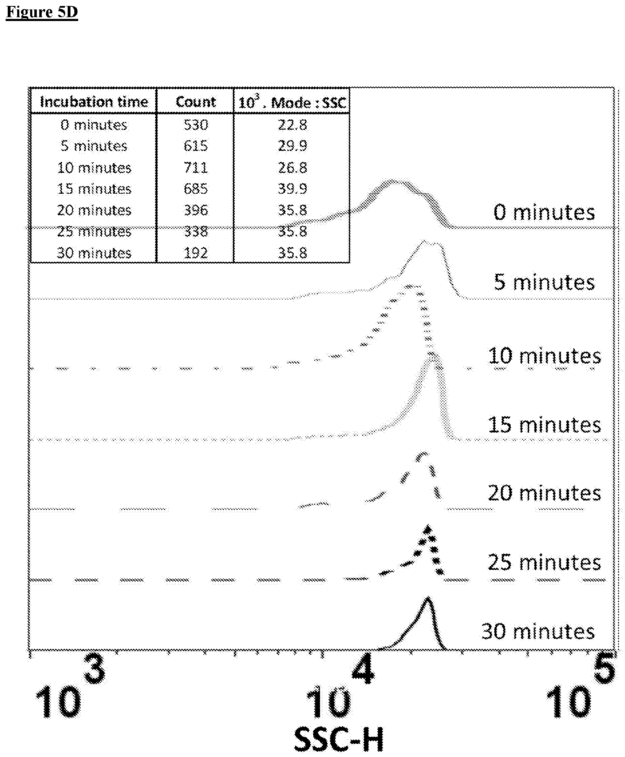

Figure 5. Side scatter histograms of the experiment mentioned in Figure 4. Ampillicin was either absent (**A,C**) or present (**B,D**) from resistant strain 16 (**A,B**) or sensitive strain 7 (**C,D**).

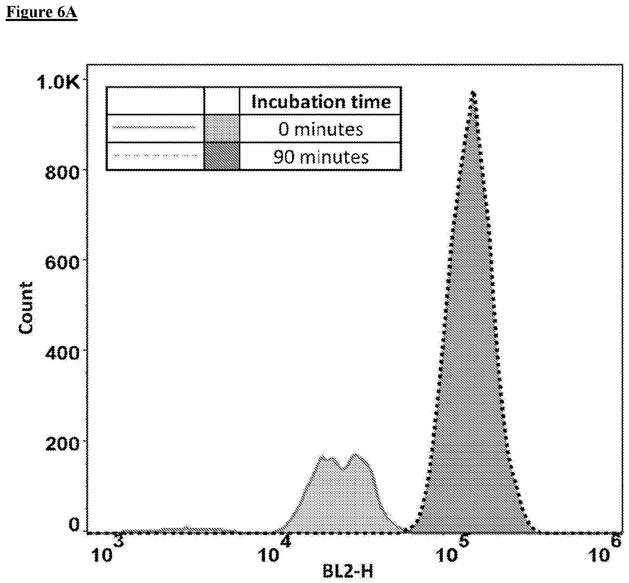

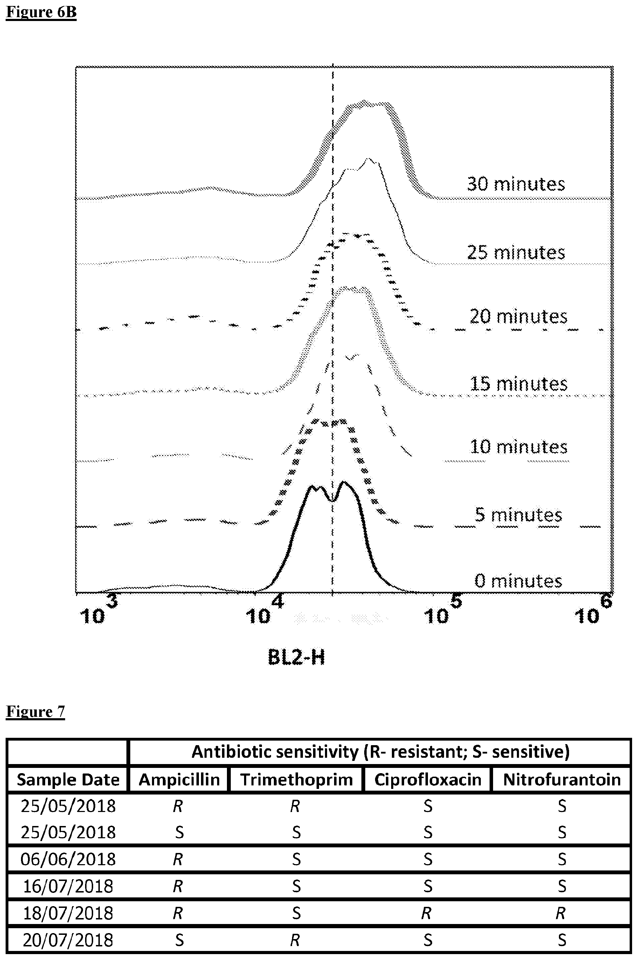

Figure 6. Effect of nitrofurantoin at 3x nominal MIC on the growth and flow cytometric behaviour of a sensitive strain of E. coli. **A,B** for nitrofurantoin, cytograms of (**A**) side scatter, (**B**) RL1 fluorescence. Experiments were performed precisely as shown in the legend to Fig 3. (**C**) Ability of flow cytometric particle counting (gated as in Fig 2) to determine the sensitivity of *E. coli* MG1655 to four different antibiotics in 20 mins.

Figure 7. DNA distributions in different populations. (**A**). DNA distributions in stationary phase (red) and exponentially growing cells (blue). The overlay histogram shows data from *E. coli* samples that were fixed with 70% ice cold ethanol and then stained using Mithramycin and Ethidium Bromide as described in Materials and Methods. The relative intensity of the BL2 channel fluorescence (488nm excitation, $572\pm14$ nm emission) shows the amount of chromosomes in the cells. The points I, II and III represent one, two and eight chromosome equivalents, respectively. The peak values of BL2 fluorescence for the points are I ($2.03.10^4$), II ($3.90.10^4$) and III ($1.53.10^5$). The cells in stationary phase (2-4h) were taken and fixed immediately while the exponentially growing cells were incubated for 90 minutes at 37°C before fixing the cells. (**B**). Changes in DNA distribution in *E. coli* cells following inoculation from a stationary phase into Terrific Broth every 5 min until 30 min. Experiments were otherwise performed exactly as described in the legend to in Figure 2.except that (to avoid spectral interference) carbocyanine was not present.

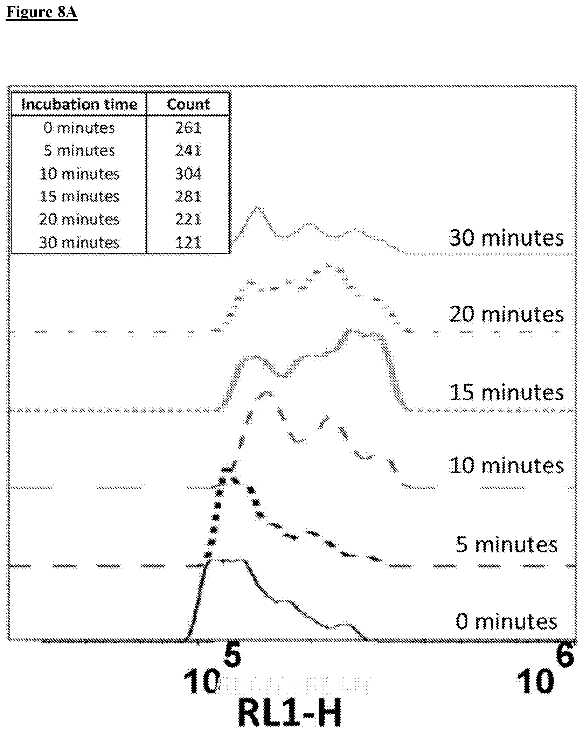

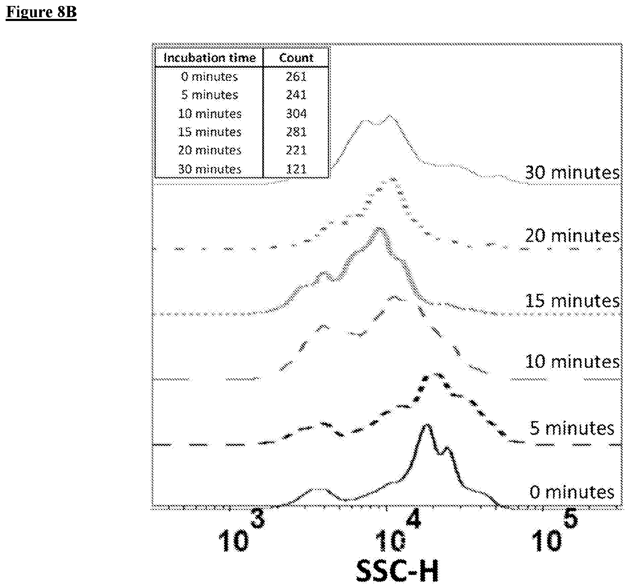

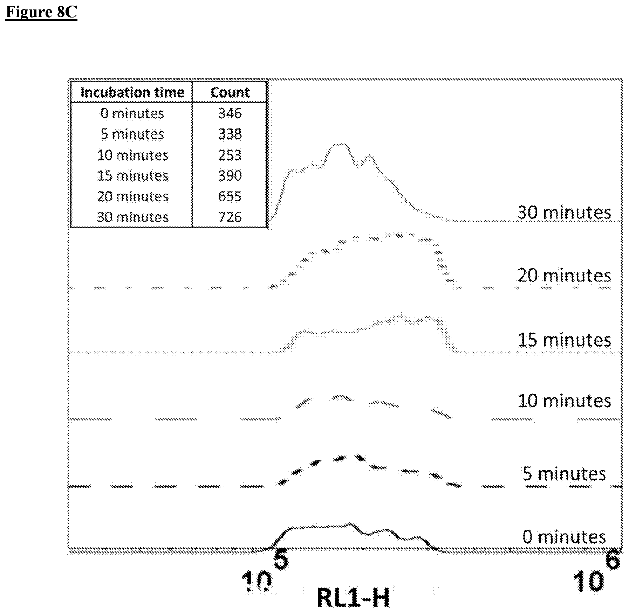

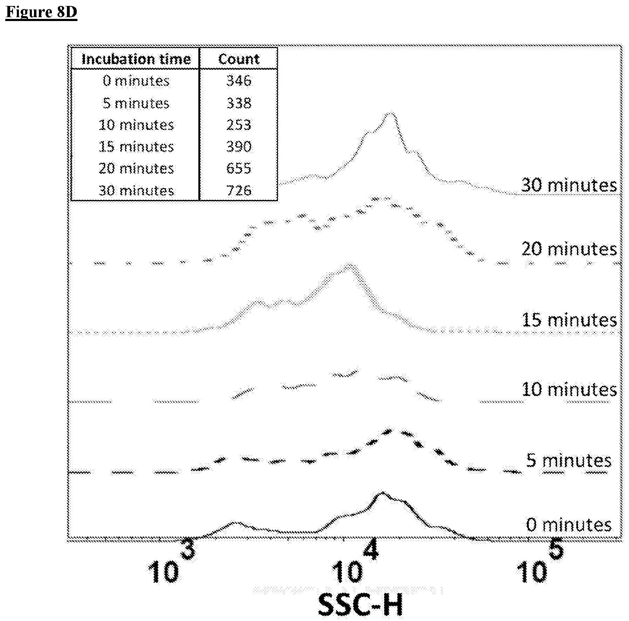

Figure 8. Cytograms of a sensitive UTI strain treated with nitrofurantoin. UTI samples (in this case containing ~ $10^6$ cells.mL$^{-1}$) were taken directly from storage, diluted tenfold into 37°C Terrific Broth including 3 μM diS-C3(5) and nitrofurantoin at a nominal 3x MIC, and measured flow cytometrically as described in the legend to Fig 2. (**A**) side scatter, (**B**) red fluorescence.

**Materials and methods**

**Microbial strains.**

*E. coli* MG1655 and a series of sensitive and resistant strains were taken from a laboratory collection.

**Culture**

*E. coli* strains were grown from inocula of appropriate concentrations in conical flasks using Lysogeny Broth to an optical density (600nm) of 1.5 - 2, representing stationary phase in this medium. They were held in stationary phase for 2-4h before being inoculated at concentration of $10^5$ cells.mL$^{-1}$ (or as noted) into Terrific Broth (Tartof and Hobbs 1987). We did not here study cells held in a long stationary phase (Finkel 2006; Navarro Llorens et al. 2010) (exceeding 3d).

**Assessment of growth by bulk OD measurements**

Bulk OD measurements were performed in 96-well plates and read at 600nm as per the manufacturer's instructions in an Omega plate reader spectrophotometer (BMG Labtech, UK) instrument. The 'background' due to scattering from the plates, etc., was not subtracted.

**Flow cytometry**

Initial studies used a Sony SH-800 instrument, but all studies reported here used an Intellicyt® iQue screener PLUS. This instrument is based in significant measure on developments by Sklar and colleagues (e.g. (Edwards et al. 2009; Sklar et al. 2007; Tegos et al. 2014)), and uses segmented flow (Skeggs 1957) to sample from 96- or 384-well U- or V-bottom plates prior to their analysis. The iQue Plus contains three excitation sources (405nm, 488nm, 640nm) and 7 fixed filter detectors (with a midpoint/range in nm of 445/45, 530/30, 572/28, 585/40, 615/24, 675/30, 780/60, giving 13 fluorescence channels) whose outputs are stored as both 'height' and area, using the FCS3.0 data file standard (Seamer et al. 1997). Forward and side scatter are obtained from the 488nm excitation source. Detection channels are referred to by the laser used (405nm violet VL, 488 nm blue BL, and 640 nm red RL) and the detector number in order of possible detectors with a longer wavelength. Thus RL1, as used for detecting di-S-C3(5), implies the red laser and the 675/30 detector. Data are collected from all channels, using a dynamic range of 7 logs. Many parameters may be used to vary the precise performance of the instrument. Those we found material to provide the best reproducibility and to minimise carryover, and their selected values, are as follows: Automatic prime - 60 secs (in Qsol buffer); Pre-plate shake - 15 s and 1500rpm; Sip time - 2 s (actual sample uptake); Additional sip time - 0.5 s (the gap between sips); Pump speed - 29 rpm (1.5 $\mu$L.s$^{-1}$ sample uptake); Plate model - U-bottom well plate (for 96 well plates); Mid plate cleanup - After every well (4 washes; 0.5 s each in Qsol buffer); Inter-well shake - 1500 rpm; after 6 wells, 4 sec in Qsol buffer; Flush and Clean - 30 sec with Decon and Clean buffers followed by 60 sec with deionised water. The Forecyt ™ software supplied with the instrument may be used to gate and display all the analyses post hoc. It, and the FlowJo software, were used in the preparation of the cytograms shown. Where used, di-S-C3(5) was present at a final concentration of 3 $\mu$M; its analysis used excitation at 640 nm and detection at $675\pm15$ nm, the fluorescence channel being referred to as RL1. For DNA analyses, cells were fixed by injection into ice-cold ethanol (final concentration 70%), washed twice by centrifugation in 0.1 M-Tris/HCl buffer, pH 7.4, before resuspension in the same buffer containing mithramycin (50 $\mu$g mL$^{-1}$) and ethidium bromide (25 $\mu$g mL$^{-1}$), MgCl$_2$, (25 mM) and NaCl (100 mM) (Boye et al. 1983). Under these circumstances, the excitation energy absorbed by mithramycin (excitation 405 or 488nm) is transferred to the ethidium bromide, providing a large Stokes shift (emission at 572, 585 or 615 nm; we chose 572 nm as it provided the best signal) and high selectivity for DNA (as mithramycin does not bind to RNA). All the solutions and media used were filtered through 0.2 $\mu$m filter.

**UTI samples**

Following ethics approval from the University of Manchester and the obtaining of signed consent forms, patients attending the Firsway clinic with suspected UTI were offered to opportunity to have their urine samples analysed by our method as well as the reference method used in a centralised pathology laboratory. Samples were taken at various times during the day, kept at 4°C, delivered to the Manchester laboratory by taxi, plated out (LB agar containing as appropriate the stated antibiotics at 3 times normal MIC) to assess microbial numbers and antibiotic sensitivity, the remaining sample kept again at 4°C, and analysed flow cytometrically within 18h. For flow cytometric assessment, cells were diluted into 37°C Terrific Broth containing 3 $\mu$M diS-C3(5) plus any appropriate antibiotic, and assayed as above.

For other experiments (not shown) cells were filtered (0.45 $\mu$m) and diluted as appropriate into warmed terrific broth. No significant differences were discernible in the two methods.

### Reagents

All reagents were of analytical grade where available. Flow cytometric dyes were obtained from AAT Bioquest.

### Results

### Initial assessment of regrowth by bulk light scattering measurements in 96-well plates

Figure 1 shows a typical lag phase from an inoculum of $10^5$ cells.mL$^{-1}$ that had spent 4h in stationary phase when inoculated into Terrific Broth (Tartof and Hobbs 1987) as observed by bulk OD measurements. For strain MG1655 it amounts to some 230 min, while it is lower (2.5-3h) for the more virulent clinical isolates (not shown). A rule of thumb states that an OD of 1 is approximately equal to 0.5 mg.mL$^{-1}$ dry weight bacteria or $\sim 10^9$.cells.mL$^{-1}$ for *E. coli.* Thus, the change in OD if $10^5$ cells.mL$^{-1}$ increase their number by 50% is ~0.00015, which is immeasurably small in this instrument. Given the noise in the system (probably mainly due to fluctuations in the incident light intensity), it is reasonable that we might, in this system, detect changes in OD of 0.01 ($\sim 10^7$ cells.mL$^{-1}$), which requires a 100-fold increase in cell number over the inoculum (~7 doublings). With a true lag phase of 10-15 min, and a doubling time of 20 min, this is indeed roughly what can be observed (Figure 1)(see also (Chandra and Singh 2018; Pin and Baranyi 2006, 2008)). When samples were taken from the same strain and plated out to estimate proliferation by CFU, the results were indeed equally consistent with those at the longer times (Figure 1).

### Flow cytometric assessment of cells and cell proliferation

Figure 2A shows a typical set of traces of multiple wells from the Intellicyt iQue, each containing an inoculum of $10^5$ cells.mL$^{-1}$. Each analysis is of 3 $\mu$L (taking 2s), and the good reproducibility is evident, especially in the inset stacked plot. We also show (rear trace) the cytograms of a bead cocktail; this Figure 2B shows that the distribution in cell properties is significantly greater than that of beads, and its significant width is thus not due to any inadequacies in the detector. The quality of a 'high-throughput' (or indeed any other) assay is nowadays widely assessed using the Z' statistic (Zhang et al. 1999). This is given, for an assay in which the sample's readout exceeds that of the control, as:

$$Z' = 1 - 3(SD\ Sample + SD\ control)/(mean\ of\ sample - mean\ of\ control)\ Eq.\ 1.$$

It is normally considered (Zhang et al. 1999) that a Z' factor exceeding 0.5 provides for a satisfactory assay.

Figures 2C and 2D show the full cytograms for forward scatter and side scatter, respectively vs RL1, illustrating the amount of small particulates remaining in terrific broth, despite extensive filtering. Consequently, we used a series of gates to assess solely the bacteria in our samples. These are shown in Figure 2E.

Figure 3 shows cytograms at various times after inoculation of the stationary phase (LB-grown) cells into Terrific Broth, along with labels of cell numbers within the regions of interest. These allow the assessment of the Z' values as per equation 1. From Fig 3B it may be observed that Z' > 0.5 from as early as 20 min, this then representing the earliest that we can robustly detect proliferation. Changes in cell constitution as judged by light scatter can, however, be detected from the earliest time point (5 min, Fig 3A top left). It is noteworthy that the proliferation (as measured by the increase in cell numbers on the ordinate) is parallelled, at least initially, by an increase in uptake of the carbocyanine dye (on the abscissa); as the cells 'wake up' they become increasingly energised, until they settle down (also observed via side scatter). For a lower concentration of starting inoculum ($5 \times 10^4$ cells.mL$^{-1}$), the Z' > 0.5 from 25 min as shown in figure 3C.

### Flow cytometric assessment of antibiotic sensitivity

Figure 4 shows similar data for a resistant (Fig 4A,B) and a sensitive strain (Fig 4C,D) in the absence ((4A,C) and presence (Fig 4B,D) of the antibiotic ampicillin, applied at three times the known MIC (MIC = 32 mg.L$^{-1}$) (http://www.eucast.org/fileadmin/src/media/PDFs/EUCAST_files/Breakpoint_tables/v_8.1_Breakpoint_Tables.pdf) (The European Committee on Antimicrobial Susceptibility Testing. Breakpoint tables for interpretation of MICs and zone diameters. Version 8.1, 2018. http://www.eucast.org.). It is clear that the susceptible strain differs (and thereby can be discriminated) from the resistant strain in at least three ways: (i) the kinetics of changes in cell numbers as judged by RL1 counts, (ii) the same as judged by forward (not shown) or side scatter, (iii) kinetic changes in the magnitude of the fluorescence.

Since we had seen rapid changes in side scatter within 5 min (Figure 3) it was also of interest to study this as a means of detecting antibiotic sensitivity. Figure 5 shows that the changes in side scatter also differs noticeably between sensitive and resistant strains in 5-10 minutes, albeit that limited proliferation was taking place.

Of course different antibiotics have different modes of action (Brochado et al. 2018; Zampieri et al. 2018), and the optimal readout needs to reflect this. Thus, nitrofurantoin is widely prescribed for UTIs and its effects on our standard laboratory system are shown in Fig 6A,B (cytograms of side scatter and RL1, respectively). The effects on cell proliferation of nitrofurantoin and several other antibiotics are given in Fig 6C. Note that the initial and later cell numbers for nitrofurantoin appear lower because this antibiotic absorbs light at the excitation wavelength (its peak is at 620 nm). Both the bacteriostatic (trimethoprim) and bactericidal (ampicillin, ciprofloxacin, nitrofurantoin) antibiotics can be seen to work effectively on this sensitive strain.

**Flow cytometric assessment of DNA distributions**

Another important strategy for detecting bacteria uses their DNA (e.g. (Hammes and Egli 2010; Jernaes and Steen 1994; Müller and Nebe-von-Caron 2010)). Thus, another high-level guide to the physiology of *E. coli* cells and cultures is the flow cytometrically observable distribution of DNA therein, as this can vary widely as a function of growth substrate, temperature, and during the cell cycle (Boye and Lobner-Olesen 1991; Skarstad et al. 1986; Skarstad et al. 1985; Steen and Boye 1980; Stokke et al. 2012). Specifically, the solution to the problem that DNA replication rates are fixed while growth rates can both vary and exceed them is to allow multiple replication forks in a given cell (Cooper and Helmstetter 1968). To this end, we compared the DNA distributions of our cultures under various conditions. Fig 7A shows both stationary phase and exponentially growing cells stained with a mithramycin-ethidium bromide cocktail as per the protocol of Skarstad and colleagues given in Materials and Methods. As they have previously observed (Boye et al. 1983; Skarstad et al. 1985), (very slowly growing or) stationary phase cells display either one or two chromosome complements, while those growing exponentially in lysogeny broth (LB medium) can have as many as eight or more chromosomes. This is entirely consistent with the basic and classical Cooper-Helmstetter model (Cooper and Helmstetter 1968) and more modern refinements (Sauls et al. 2016; Si et al. 2017; Willis and Huang 2017; Zheng et al. 2016). To this end, Fig 7B shows changes in the DNA distribution of cells taken from a similar regrowth experiment to that in Fig 2. It is evident that both the one- and two-chromosome-containing cells from the stationary phase initiate increases in their DNA content on the same kinds of timescale as may be observed from both direct cell counting (proliferation) and carbocyanine fluorescence, with the initially bimodal DNA distribution morphing into a more monomodal one. This implies that the initial increase in cell numbers over 15 min or so involves cells that were about to divide actually dividing, and provides another useful metric of cellular (cell cycling) activity, albeit one that requires sampling as the cells must be permeabilised, at least for this protocol.

**Flow cytometric analysis of UTI samples**

Finally, we wished to determine whether this method, as developed in laboratory cultures, could be applied to candidate UTI specimens 'as received' in a doctor's surgery. To this end, we analysed 23 samples, of which six were in fact positive as judged by a reference method performed in a central microbiology laboratory. Each of these was also found to be positive using our methods, and with the antibiotic sensitivities given in Table 1 below. These were again consistent with the reference method.

Table 1. Antibiotic sensitivity profile for the six positive samples (taken to be $\geq 10^5.mL^{-1}$) obtained from the Firsway clinic.

| Sample Date | Antibiotic sensitivity (R- resistant; S- sensitive) | | | |
|---|---|---|---|---|
| | **Ampicillin** | **Trimethoprim** | **Ciprofloxacin** | **Nitrofurantoin** |
| 25/05/2018 | *R* | *R* | S | S |
| 25/05/2018 | S | S | S | S |
| 06/06/2018 | *R* | S | S | S |
| 16/07/2018 | *R* | S | S | S |
| 18/07/2018 | *R* | S | *R* | *R* |
| 20/07/2018 | S | *R* | S | S |

Typical cytograms for sensitive and resistant strains are given in Fig 8. The positive cultures were speciated centrally, and in each case the organism was found to be *E. coli.*

## Discussion and conclusions

It is often considered that the 'lag' phase of bacterial growth is one in which very little is happening, and that what is happening is happening quite slowly. This notion probably stems from the fact that changes in OD observable by the naked eye in laboratory cultures (Kaprelyants and Kell 1993) are indeed quite sluggish. However, the very few papers that have studied this in any detail (Baltekin et al. 2017; Madar et al. 2013; Novotna et al. 2003; Pin et al. 2009; Rolfe et al. 2012; Roostalu et al. 2008; Schoepp et al. 2017; Yu et al. 2018) have found that changes in expression profiles (albeit mainly measured at a bulk level) actually occur on a very rapid timescale indeed, possibly in 4 minutes or less following reinoculation into a rich growth medium. For antibiotics to have an observable, and in terms of sensitivity to them a differentially observable, effect on cells, the cells need to be in a replicative state. This might be thought to preclude any such observations in the lag phase, but what is clear from the present observations is that cells can re-initiate or continue their cell cycles very rapidly, such that observable proliferation can occur in as little as 15-20 min after reinoculation of starved, stationary phase cells into rich medium. Consequently it is not necessary to wait for a full period of 'lag-plus-first-division time' (Baltekin et al. 2017), which can be well over one hour (Pin and Baranyi 2006, 2008). The rapid proliferation that we describe could be observed by light scattering, by cell counting, by carbocyanine fluorescence (membrane energisation), and by changes in the magnitude and distribution of DNA in the population. This has allowed us to determine, using any or all of these phenotypic assays, antibiotic susceptibility at a phenotypic level in what would appear to be a record time. Pin and Baranyi (Pin and Baranyi 2006, 2008) observed a more stochastic and somewhat slower process than that which we observed here, but in their case they were measuring CFU only, and the inoculation was into the less rich LB, while we used Terrific Broth. Indeed, the exit from lag phase can be very heterogeneous when organisms are measured individually (Aguirre et al. 2013; Aguirre and Koutsoumanis 2016; Baltekin et al. 2017; Stylianidou et al. 2016).

While we did not study this at the level of the transcriptome here, the dynamics of the physiological changes observed during the early lag and regrowth phases as observed by the uptake of the carbocyanine dye are of interest. Classically, its uptake has been considered to reflect a transmembrane potential difference (negative inside) (e.g. (Bashford 1981; Ghazi et al. 1981; Johnson et al. 1981; Shapiro 2000; Waggoner 1976; Waggoner 1979), but cf. (Felle et al. 1978)) based on bilayer-mediated equilibration according to the Nernst equation (Rottenberg 1979). However, we recognise that such cyanine dyes, much as ethidium bromide (Jernaes and Steen 1994) and other xenobiotics (Kell et al. 2013; Kell and Oliver 2014), are likely to be both influx and efflux substrates for various transporters (Wu et al. 2015), so such an interpretation should be treated with some caution.

A similar strategy may usefully be applied to other cells (including pathogens in more difficult matrices such as urine), other antibiotics and other stains. However, the present work provides a very useful springboard for these by showing that one may indeed expect to be able to determine antibiotic susceptibility in a phenotypic assay in 20 minutes or less. This could be a very useful attribute in the fight against anti-microbial resistance.

## References

Aguirre JS, Gonzalez A, Ozcelik N, Rodriguez MR, Garcia de Fernando GD: Modeling the Listeria innocua micropopulation lag phase and its variability. Int J Food Microbiol 2013; 164:60-69.

Aguirre JS, Koutsoumanis KP: Towards lag phase of microbial populations at growth-limiting conditions: The role of the variability in the growth limits of individual cells. Int J Food Microbiol 2016; 224:1-6.

Åkerlund T, Nordström K, Bernander R: Analysis of cell size and DNA content in exponentially growing and stationary-phase batch cultures of Escherichia coli. J Bacteriol 1995; 177:6791-6797.

Álvarez-Barrientos A, Arroyo J, Cantón R, Nombela C, Sánchez-Pérez M: Applications of flow cytometry to clinical microbiology. Clin Microbiol Rev 2000; 13:167-195.

Andersson DI, Hughes D: Antibiotic resistance and its cost: is it possible to reverse resistance? Nat Rev Microbiol 2010; 8:260-271.

Baker S, Thomson N, Weill FX, Holt KE: Genomic insights into the emergence and spread of antimicrobial-resistant bacterial pathogens. Science 2018; 360:733-738.

Baltekin Ö, Boucharin A, Tano E, Andersson DI, Elf J: Antibiotic susceptibility testing in less than 30 min using direct single-cell imaging. Proc Natl Acad Sci U S A 2017; 114:9170-9175.

Baranyi J, Pin C: Estimating bacterial growth parameters by means of detection times. Appl Environ Microbiol 1999; 65:732-736.

Baranyi J, Roberts TA: A dynamic approach to predicting bacterial growth in food. Int J Food Microbiol 1994; 23:277-294.

Bashford CL: The measurement of membrane potential using optical indicators. Biosci Rep 1981; 1:183-196.

Baty F, Delignette-Muller ML: Estimating the bacterial lag time: which model, which precision? Int J Food Microbiol 2004; 91:261-277.

Baty F, Flandrois JP, Delignette-Muller ML: Modeling the lag time of Listeria monocytogenes from viable count enumeration and optical density data. Appl Environ Microbiol 2002; 68:5816-5825.

Bertrand RL: Lag phase-associated iron accumulation is likely a microbial counter-strategy to host iron sequestration: role of the ferric uptake regulator (fur). J Theor Biol 2014; 359:72-79.

Boi P, Manti A, Pianetti A, Sabatini L, Sisti D, Rocchi MB, Bruscolini F, Galluzzi L, Papa S: Evaluation of Escherichia coli viability by flow cytometry: A method for determining bacterial responses to antibiotic exposure. Cytometry B Clin Cytom 2015; 88:149-153. Boye E, Løbner-Olesen A: Bacterial Growth Control Studied by Flow Cytometry. Res Microbiol 1991; 142:131-135.

Boye E, Steen HB, Skarstad K: Flow Cytometry of Bacteria: A Promising Tool in Experimental and Clinical Microbiology. J Gen Microbiol 1983; 129:973-980.

Brochado AR, Telzerow A, Bobonis J, Banzhaf M, Mateus A, Selkrig J, Huth E, Bassler S, Zamarreno Beas J, Zietek M, Ng N, Foerster S, Ezraty B, Py B, Barras F, Savitski MM, Bork P, Göttig S, Typas A: Species-specific activity of antibacterial drug combinations. Nature 2018; 559:259-263.

Bryce A, Hay AD, Lane IF, Thornton HV, Wootton M, Costelloe C: Global prevalence of antibiotic resistance in paediatric urinary tract infections caused by Escherichia coli and association with routine use of antibiotics in primary care: systematic review and meta-analysis. BMJ 2016; 352:i939.

Buchan BW, Ledeboer NA: Emerging technologies for the clinical microbiology laboratory. Clin Microbiol Rev 2014; 27:783-822.

Cek M, Tandogdu Z, Wagenlehner F, Tenke P, Naber K, Bjerklund-Johansen TE: Healthcare-associated urinary tract infections in hospitalized urological patients--a global perspective: results from the GPIU studies 2003-2010. World J Urol 2014; 32:1587-1594. Chandra A, Singh N: Bacterial growth sensing in microgels using pH-dependent fluorescence emission. Chem Commun (Camb) 2018; 54:1643-1646.

Chien TI, Kao JT, Liu HL, Lin PC, Hong JS, Hsieh HP, Chien MJ: Urine sediment examination: a comparison of automated urinalysis systems and manual microscopy. Clin Chim Acta 2007; 384:28-34.

Choi J, Yoo J, Lee M, Kim EG, Lee JS, Lee S, Joo S, Song SH, Kim EC, Lee JC, Kim HC, Jung YG, Kwon S: A rapid antimicrobial susceptibility test based on single-cell morphological analysis. Sci Transl Med 2014; 6:267ra174.

Coates AR, Halls G, Hu Y: Novel classes of antibiotics or more of the same? Br J Pharmacol 2011; 163:184-194.

Cooper S, Helmstetter CE: Chromosome Replication and the Division Cycle of Escherichia coli B/r. J Mol Biol 1968; 31:519-540.

Dalgaard P, Ross T, Kamperman L, Neumeyer K, McMeekin TA: Estimation of bacterial growth rates from turbidimetric and viable count data. Int J Food Microbiol 1994; 23:391-404.

Davey HM: Life, death, and in-between: meanings and methods in microbiology. Appl Environ Microbiol 2011;

77:5571-5576.

Davey HM, Kell DB: Flow cytometry and cell sorting of heterogeneous microbial populations: the importance of single-cell analysis. Microbiol Rev 1996; 60:641-696.

Detweiler K, Mayers D, Fletcher SG: Bacteruria and urinary tract infections in the elderly. Urol Clin North Am 2015; 42:561-568.

Didelot X, Bowden R, Wilson DJ, Peto TEA, Crook DW: Transforming clinical microbiology with bacterial genome sequencing. Nat Rev Genet 2012; 13:601-612.

Du D, Wang-Kan X, Neuberger A, van Veen HW, Pos KM, Piddock LJV, Luisi BF: Multidrug efflux pumps: structure, function and regulation. Nat Rev Microbiol 2018.

Dunne WM, Jr., Jaillard M, Rochas O, Van Belkum A: Microbial genomics and antimicrobial susceptibility testing. Expert Rev Mol Diagn 2017; 17:257-269.

Edwards BS, Young SM, Ivnitsky-Steele I, Ye RD, Prossnitz ER, Sklar LA: High-content screening: flow cytometry analysis. Methods Mol Biol 2009; 486:151-165.

Ejrnaes K: Bacterial characteristics of importance for recurrent urinary tract infections caused by Escherichia coli. Dan Med Bull 2011; 58: B4187.

Farha MA, Brown ED: Chemical probes of Escherichia coli uncovered through chemical-chemical interaction profiling with compounds of known biological activity. Chem Biol 2010; 17:852-862.

Felle H, Stetson DL, Long WS, Slayman CL: Direct measurement of membrane potential and resistance in giant cells of Escherichia colil. Front Biol Energet 1978; 2:1399-1407. Finkel SE: Long-term survival during stationary phase: evolution and the GASP phenotype. Nat Rev Microbiol 2006; 4:113-120.

Foxman B: The epidemiology of urinary tract infection. Nat Rev Urol 2010; 7:653-660. Gant VA, Warnes G, Phillips I, Savidge GF: The application of flow cytometry to the study of bacterial responses to antibiotics. J Med Microbiol 1993; 39:147-154.

Gelband H, Laxminarayan R: Tackling antimicrobial resistance at global and local scales. Trends Microbiol 2015; 23:524-526.

Ghazi A, Schechter E, Letellier L, Labedan B: Probes of membrane potential in Escherichia coli cells. FEBS Lett 1981; 125:197-200.

Hammes F, Egli T: Cytometric methods for measuring bacteria in water: advantages, pitfalls and applications. Anal Bioanal Chem 2010; 397:1083-1095.

Hewitt CJ, Nebe-Von-Caron G: The application of multi-parameter flow cytometry to monitor individual microbial cell physiological state. Adv Biochem Eng Biotechnol 2004; 89:197-223.

Himeoka Y, Kaneko K: Theory for Transitions Between Exponential and Stationary Phases: Universal Laws for Lag Time. Phys Rev X 2017; 7.

Hong W, Karanja CW, Abutaleb NS, Younis W, Zhang X, Seleem MN, Cheng JX: Antibiotic susceptibility determination within one cell cycle at single-bacterium level by stimulated Raman metabolic imaging. Anal Chem 2018; 90:3737-3743.

Iyer R, Ferrari A, Rijnbrand R, Erwin AL: A fluorescent microplate assay quantifies bacterial efflux and demonstrates two distinct compound binding sites in AcrB. Antimicrob Agents Chemother 2015; 59:2388-2397.

Jernaes MW, Steen HB: Staining of Escherichia coli for flow cytometry: influx and efflux of ethidium bromide. Cytometry 1994; 17:302-309.

Jõers A, Tenson T: Growth resumption from stationary phase reveals memory in Escherichia coli cultures. Sci Rep 2016; 6:24055.

Johnson LV, Walsh ML, Bockus BJ, Chen LB: Monitoring of relative mitochondrial membrane potential in living cells by fluorescence microscopy. J Cell Biol 1981; 88:526-535.

Kaprelyants AS, Kell DB: Dormancy in stationary-phase cultures of Micrococcus luteus: flow cytometric analysis of starvation and resuscitation. Appl Env Microbiol 1993; 59:3187-3196.

Kaprelyants AS, Kell DB: Rapid assessment of bacterial viability and vitality using rhodamine 123 and flow cytometry. J Appl Bacteriol 1992; 72:410-422.

Kell DB, Dobson PD, Bilsland E, Oliver SG: The promiscuous binding of pharmaceutical drugs and their transporter-mediated uptake into cells: what we (need to) know and how we can do so. Drug Disc Today 2013; 18:218-239.

Kell DB, Kaprelyants AS, Weichart DH, Harwood CL, Barer MR: Viability and activity in readily culturable bacteria: a review and discussion of the practical issues. Antonie van Leeuwenhoek 1998; 73:169-187.

Kell DB, Oliver SG: How drugs get into cells: tested and testable predictions to help discriminate between transporter-mediated uptake and lipoidal bilayer diffusion. Front Pharmacol 2014; 5:231.

Kell DB, Potgieter M, Pretorius E: Individuality, phenotypic differentiation, dormancy and 'persistence' in culturable bacterial systems: commonalities shared by environmental, laboratory, and clinical microbiology. F1000Res 2015; 4:179.

Kell DB, Ryder HM, Kaprelyants AS, Westerhoff HV: Quantifying heterogeneity: Flow cytometry of bacterial cultures. Antonie van Leeuwenhoek 1991; 60:145-158.

Kelley SO: New technologies for rapid bacterial identification and antibiotic resistance profiling. SLAS Technol 2017; 22:113-121.

Kerremans JJ, Verboom P, Stijnen T, Hakkaart-van Roijen L, Goessens W, Verbrugh HA, Vos MC: Rapid identification and antimicrobial susceptibility testing reduce antibiotic use and accelerate pathogen-directed antibiotic use. J Antimicrob Chemother 2008; 61:428-435.

Kessel D, Beck WT, Kukuruga D, Schulz V: Characterization of multidrug resistance by fluorescent dyes. Cancer Res 1991; 51:4665-4670.

Kirchhoff J, Glaser U, Bohnert JA, Pletz MW, Popp J, Neugebauer U: Simple Ciprofloxacin Resistance Test and Determination of Minimal Inhibitory Concentration within 2 h Using Raman Spectroscopy. Anal Chem 2018; 90:1811-1818.

Kirkup BC, Mahlen S, Kallstrom G: Future-generation sequencing and clinical microbiology. Clinics in laboratory medicine 2013; 33:685-704.

Kline KA, Lewis AL: Gram-Positive Uropathogens, Polymicrobial Urinary Tract Infection, and the Emerging Microbiota of the Urinary Tract. Microbiology spectrum 2016; 4.

Kohanski MA, Dwyer DJ, Hayete B, Lawrence CA, Collins JJ: A common mechanism of cellular death induced by bactericidal antibiotics. Cell 2007; 130:797-810.

Koken T, Aktepe OC, Serteser M, Samli M, Kahraman A, Dogan N: Determination of cutoff values for leucocytes and bacteria for urine flow cytometer (UF-100) in urinary tract infections. Int Urol Nephrol 2002; 34:175-178.

Köser CU, Ellington MJ, Cartwright EJ, Gillespie SH, Brown NM, Farrington M, Holden MT, Dougan G, Bentley SD, Parkhill J, Peacock SJ: Routine use of microbial whole genome sequencing in diagnostic and public health microbiology. PLoS pathogens 2012; 8:e1002824.

Köves B, Cai T, Veeratterapillay R, Pickard R, Seisen T, Lam TB, Yuan CY, Bruyere F, Wagenlehner F, Bartoletti R, Geerlings SE, Pilatz A, Pradere B, Hofmann F, Bonkat G, Wullt B: Benefits and Harms of Treatment of Asymptomatic Bacteriuria: A Systematic Review and Meta-analysis by the European Association of Urology Urological Infection Guidelines Panel. Eur Urol 2017.

Kwong JC, McCallum N, Sintchenko V, Howden BP: Whole genome sequencing in clinical and public health microbiology. Pathology 2015; 47:199-210.

Laxminarayan R, Sridhar D, Blaser M, Wang M, Woolhouse M: Achieving global targets for antimicrobial resistance. Science 2016; 353:874-875.

Li B, Qiu Y, Shi H, Yin H: The importance of lag time extension in determining bacterial resistance to antibiotics. Analyst 2016; 141:3059-3067.

Link H, Fuhrer T, Gerosa L, Zamboni N, Sauer U: Real-time metabolome profiling of the metabolic switch between starvation and growth. Nat Methods 2015; 12:1091-1097.

Macedo RS, Onita JH, Wille MP, Furtado GH: Pharmacokinetics and pharmacodynamics of antimicrobial drugs in intensive care unit patients. Shock 2013; 39 Suppl 1:24-28.

Madar D, Dekel E, Bren A, Zimmer A, Porat Z, Alon U: Promoter activity dynamics in the lag phase of Escherichia coli. BMC Syst Biol 2013; 7:136.

Mason DJ, Allman R, Stark JM, Lloyd D: Rapid Estimation of Bacterial Antibiotic Susceptibility With Flow- Cytometry. Journal of Microscopy-Oxford 1994; 176:8-16.

Mason DJ, Power EGM, Talsania H, Phillips I, Gant VA: Antibacterial action of ciprofloxacin. Antimicrob Agents Ch 1995; 39:2752-2758.

Mehnert-Kay SA: Diagnosis and management of uncomplicated urinary tract infections. American Family Physician 2005; 72:451-456.

Mendelson M, Balasegaram M, Jinks T, Pulcini C, Sharland M: Antibiotic resistance has a language problem. Nature 2017; 545:23-25.

Mody L, Juthani-Mehta M: Urinary tract infections in older women: a clinical review. JAMA 2014; 311:844-854.

Müller S, Losche A, Bley T: Staining procedures for flow cytometric monitoring of bacterial populations. Acta Biotechnol 1993; 13:289-297.

Müller S, Nebe-von-Caron G: Functional single-cell analyses: flow cytometry and cell sorting of microbial populations and communities. FEMS Microbiol Rev 2010; 34:554-587.

Murray C, Adeyiga O, Owsley K, Di Carlo D: Research highlights: microfluidic analysis of antimicrobial susceptibility. Lab Chip 2015; 15:1226-1229.

Navarro Llorens JM, Tormo A, Martinez-Garcia E: Stationary phase in gram-negative bacteria. FEMS Microbiol Rev 2010; 34:476-495.

Nebe-von Caron G, Badley RA: Viability assessment of bacteria in mixed populations using flow cytometry. J Microsc 1995; 179:55-66.

Novotna J, Vohradsky J, Berndt P, Gramajo H, Langen H, Li XM, Minas W, Orsaria L, Roeder D, Thompson CJ: Proteomic studies of diauxic lag in the differentiating prokaryote Streptomyces coelicolor reveal a regulatory network of stress-induced proteins and central metabolic enzymes. Mol Microbiol 2003; 48:1289-1303.

Pieretti B, Brunati P, Pini B, Colzani C, Congedo P, Rocchi M, Terramocci R: Diagnosis of bacteriuria and leukocyturia by automated flow cytometry compared with urine culture. Journal of clinical microbiology 2010; 48:3990-3996.

Pin C, Baranyi J: Kinetics of single cells: observation and modeling of a stochastic process. Appl Environ Microbiol 2006; 72:2163-2169.

Pin C, Baranyi J: Single-cell and population lag times as a function of cell age. Appl Environ Microbiol 2008; 74:2534-2536.

Pin C, Rolfe MD, Muñoz-Cuevas M, Hinton JCD, Peck MW, Walton NJ, Baranyi J: Network analysis of the transcriptional pattern of young and old cells of Escherichia coli during lag phase. BMC Syst Biol 2009; 3:108.

Pirt SJ: Principles of microbe and cell cultivation. London: Wiley, 1975.

Prats C, Giró A, Ferrer J, López D, Vives-Rego J: Analysis and IbM simulation of the stages in bacterial lag phase: basis for an updated definition. J Theor Biol 2008; 252:56-68.

Prats C, López D, Giró A, Ferrer J, Valls J: Individual-based modelling of bacterial cultures to study the microscopic causes of the lag phase. J Theor Biol 2006; 241:939-953.

Roach DJ, Burton JN, Lee C, Stackhouse B, Butler-Wu SM, Cookson BT, Shendure J, Salipante SJ: A year of infection in the Intensive Care Unit: prospective whole genome sequencing of bacterial clinical isolates reveals cryptic transmissions and novel microbiota. PLoS Genet 2015; 11:e1005413.

Roca I, Akova M, Baquero F, Carlet J, Cavaleri M, Coenen S, Cohen J, Findlay D, Gyssens I, Heure OE, Kahlmeter G, Kruse H, Laxminarayan R, Liébana E, López-Cerero L, MacGowan A, Martins M, Rodriguez-Bano J, Rolain JM, Segovia C, Sigauque B, Taconelli E, Wellington E, Vila J: The global threat of antimicrobial resistance: science for intervention. New Microbes New Infect 2015; 6:22-29.

Rolfe MD, Rice CJ, Lucchini S, Pin C, Thompson A, Cameron ADS, Alston M, Stringer MF, Betts RP, Baranyi J, Peck MW, Hinton JCD: Lag phase is a distinct growth phase that prepares bacteria for exponential growth and involves transient metal accumulation. J Bacterio/2012; 194:686-701.

Roostalu J, Jõers A, Luidalepp H, Kaldalu N, Tenson T: Cell division in Escherichia coli cultures monitored at single cell resolution. BMC Microbiol 2008; 8.

Rottenberg H: The measurement of membrane potential and deltapH in cells, organelles, and vesicles. Methods Enzymol 1979; 55:547-569.

Sauls JT, Li D, Jun S: Adder and a coarse-grained approach to cell size homeostasis in bacteria. Curr Opin Cell Biol 2016; 38:38-44.

Schmidt K, Mwaigwisya S, Crossman LC, Doumith M, Munroe D, Pires C, Khan AM, Woodford N, Saunders NJ, Wain J, O'Grady J, Livermore DM: Identification of bacterial pathogens and antimicrobial resistance directly from clinical urines by nanopore-based metagenomic sequencing. J Antimicr Chemother 2016.

Schmiemann G, Kniehl E, Gebhardt K, Matejczyk MM, Hummers-Pradier E: The diagnosis of urinary tract infection: a systematic review. Dtsch Ärztebl Int 2010; 107:361-367.

Schoepp NG, Schlappi TS, Curtis MS, Butkovich SS, Miller S, Humphries RM, Ismagilov RF: Rapid pathogen-specific phenotypic antibiotic susceptibility testing using digital LAMP quantification in clinical samples. Sci Transl Med 2017; 9.

Schultz D, Kishony R: Optimization and control in bacterial lag phase. BMC Biol 2013; 11:120.

Seamer LC, Bagwell CB, Barden L, Redelman D, Salzman GC, Wood JCS, Murphy RF: Proposed new data file standard for flow cytometry, version FCS 3.0. Cytometry 1997; 28:118-122.

Senyurek I, Paulmann M, Sinnberg T, Kalbacher H, Deeg M, Gutsmann T, Hermes M, Kohler T, Gotz F, Wolz C, Peschel A, Schittek B: Dermcidin-derived peptides show a different mode of action than the cathelicidin LL-37 against Staphylococcus aureus. Antimicrob Agents Chemother 2009; 53:2499-2509.

Shang YJ, Wang QQ, Zhang JR, Xu YL, Zhang WW, Chen Y, Gu ML, Hu ZD, Deng AM: Systematic review and meta-analysis of flow cytometry in urinary tract infection screening. Clin Chim Acta 2013; 424:90-95.

Shapiro HM: Flow cytometry of bacterial membrane potential and permeability. Methods Mol Med 2008; 142:175-186.

Shapiro HM: Membrane potential estimation by flow cytometry. Methods 2000; 21:271-279.

Shapiro HM: Multiparameter flow cytometry of bacteria: implications for diagnostics and therapeutics. Cytometry 2001; 43:223-226.

Shapiro HM: Practical Flow Cytometry, 4th edition. New York: John Wiley, 2003.

Shayanfar N, Tobler U, von Eckardstein A, Bestmann L: Automated urinalysis: first experiences and a comparison between the Iris iQ200 urine microscopy system, the Sysmex UF-100 flow cytometer and manual microscopic particle counting. Clin Chem Lab Med 2007; 45:1251-1256.

Si F, Li D, Cox SE, Sauls JT, Azizi O, Sou C, Schwartz AB, Erickstad MJ, Jun Y, Li X, Jun S: Invariance of Initiation Mass and Predictability of Cell Size in Escherichia coli. Curr Biol 2017; 27:1278-1287.

Skarstad K, Boye E, Steen HB: Timing of Initiation of Chromosome Replication in Individual Escherichia coli cells. EMBO Journal 1986; 5:1711-1717.

Skarstad K, Steen HB, Boye E: Escherichia coli DNA Distributions Measured by Flow Cytometry and Compared with Theoretical Computer Simulations. J Bacteriol 1985; 163:661-668.

Skeggs LT, Jr.: An automatic method for colorimetric analysis. Am J Clin Pathol 1957; 28:311-322.

Sklar LA, Carter MB, Edwards BS: Flow cytometry for drug discovery, receptor pharmacology and high-throughput screening. Curr Opin Pharmacol 2007; 7:527-534. Steen HB: Flow cytometric studies of microorganisms. In Melamed MR, Lindmo T, Mendelsohn ML (eds.): Flow Cytometry and Sorting (2nd Edition). New York: Wiley-Liss Inc., 1990:605-622.

Steen HB, Boye E: Bacterial growth studied by flow cytometry. Cytometry 1980; 1:32-36. Stokke C, Flåtten I, Skarstad K: An easy-to-use simulation program demonstrates variations in bacterial cell cycle parameters depending on medium and temperature. PLoS One 2012; 7:e30981.

Stylianidou S, Brennan C, Nissen SB, Kuwada NJ, Wiggins PA: SuperSegger: robust image segmentation, analysis and lineage tracking of bacterial cells. Mol Microbiol 2016; 102:690-700.

Swinnen IAM, Bernaerts K, Dens EJJ, Geeraerd AH, Van Impe JF: Predictive modelling of the microbial lag phase: a review. Int J Food Microbiol 2004; 94:137-159.

Taheri-Araghi S, Brown SD, Sauls JT, McIntosh DB, Jun S: Single-Cell Physiology. Annu Rev Biophys 2015; 44:123-142.

Tandogdu Z, Wagenlehner FM: Global epidemiology of urinary tract infections. Curr Opin Infect Dis 2016; 29:73-79.

Tartof KD, Hobbs CA: Improved Media for Growing Plasmid and Cosmid Clones. Bethseda Research Laboratories Focus 1987; 9:12.

Tegos GP, Evangelisti AM, Strouse JJ, Ursu O, Bologa C, Sklar LA: A high throughput flow cytometric assay platform targeting transporter inhibition. Drug Disc Today Technol 2014; 12:e95-e103.

Tsai EA, Shakbatyan R, Evans J, Rossetti P, Graham C, Sharma H, Lin CF, Lebo MS: Bioinformatics Workflow for Clinical Whole Genome Sequencing at Partners HealthCare Personalized Medicine. J Pers Med 2016; 6.

Tuite N, Reddington K, Barry T, Zumla A, Enne V: Rapid nucleic acid diagnostics for the detection of antimicrobial resistance in Gram-negative bacteria: is it time for a paradigm shift? J Antimicrob Chemother 2014; 69:1729-1733.

van Belkum A, Dunne WM, Jr.: Next-generation antimicrobial susceptibility testing. Journal of clinical microbiology 2013; 51:2018-2024.

Waggoner A: Optical probes of membrane potential. J Membr Biol 1976; 27:317-334. Waggoner AS: Dye indicators of membrane potential. Annu Rev Biophys Bioeng 1979; 8:47-68.

Walberg M, Gaustad P, Steen HB: Rapid assessment of ceftazidime, ciprofloxacin, and gentamicin susceptibility in exponentially-growing E-coli cells by means of flow cytometry. Cytometry 1997; 27:169-178.

Wallden M, Fange D, Lundius EG, Baltekin Ö, Elf J: The synchronization of replication and division cycles in individual E. coli cells. Cell 2016; 166:729-739.

Wang J, Zhang Y, Xu D, Shao W, Lu Y: Evaluation of the Sysmex UF-1000i for the diagnosis of urinary tract infection. Am J Clin Pathol 2010; 133:577-582.

Willis L, Huang KC: Sizing up the bacterial cell cycle. Nat Rev Microbiol 2017; 15:606-620. Wilson ML, Gaido L: Laboratory diagnosis of urinary tract infections in adult patients. Clin Infect Dis 2004; 38:1150-1158.

Wu JBY, Shi CH, Chu GCY, Xu QJ, Zhang Y, Li QL, Yu JS, Zhau HYE, Chung LWK: Near-infrared fluorescence heptamethine carbocyanine dyes mediate imaging and targeted drug delivery for human brain tumor. Biomaterials 2015; 67:1-10.

Yu H, Jing W, Iriya R, Yang Y, Syal K, Mo M, Grys TE, Haydel SE, Wang S, Tao N: Phenotypic antimicrobial susceptibility testing with deep learning video microscopy. Anal Chem 2018; 90:6314-6322.

Zampieri M, Szappanos B, Buchieri MV, Trauner A, Piazza I, Picotti P, Gagneux S, Borrell S, Gicquel B, Lelievre J, Papp B, Sauer U: High-throughput metabolomic analysis predicts mode of action of uncharacterized antimicrobial compounds. Sci Transl Med 2018; 10.

Zhang JH, Chung TDY, Oldenburg KR: A simple statistical parameter for use in evaluation and validation of high throughput screening assays. J Biomol Screening 1999; 4:67-73.

Zheng H, Ho PY, Jiang M, Tang B, Liu W, Li D, Yu X, Kleckner NE, Amir A, Liu C: Interrogating the Escherichia coli cell cycle by cell dimension perturbations. Proc Natl Acad Sci U S A 2016; 113:15000-15005.

## Claims

1. A method for rapidly determining the susceptibility of a microorganism in a urine sample to an antimicrobial agent comprising the steps:

   a) contacting a first sample containing the microorganism with a first growth medium so as to form a first mixture, wherein the first growth medium is selected to enable the microorganism to proliferate and/or encourage the microorganism cell cycle to commence proliferation;
   b) contacting a second sample containing the microorganism with a second growth medium so as to form a second mixture, wherein the second growth medium is substantially the same as the first growth medium but further comprises a first antimicrobial agent which may inhibit or slow the proliferation of the microorganism;
   c) incubating the first and second mixtures, for 30 minutes or less, under conditions suitable to enable or encourage proliferation of the microorganism;
   d) assessing the first and second mixture, or portion thereof, through a flow cytometer in order to assess one or more biochemical and/or biophysical parameters of the microorganisms in both mixtures; and
   e) comparing the parameters of the microorganisms in the first mixture with that of the second mixture, after incubation, in order to detect whether the first antimicrobial agent inhibits or slows the proliferation of the microorganism so as to determine the susceptibility of a microorganism to said agent,

   wherein the parameters are:

   (i) cell size, cell number and/or cell membrane energisation, and wherein prior to step d), carbocyanine dye, comprising 3,3'-dipropylthiadicarbocyanine iodide (di-S-C3(5)), is added to the mixture or part of the mixture; and (ii) nucleic acid content and/or distribution and said nucleic acid comprises DNA and wherein prior to step d), mithramycin and a nucleic acid stain, comprising SYBR Green or ethidium bromide, are added to the mixture or part of the mixture.

2. The method as claimed in claim 1, wherein step b) further comprises contacting one or more further samples containing the microorganism with a one or more further growth media so as to form one or more further mixtures, wherein the one or more further growth media is the same as the first growth medium but further comprises one or more further antimicrobial agents which may inhibit or slow the proliferation of the microorganism, wherein said one or more further antimicrobial agents are different from one another and different from the first antimicrobial agent.

3. The method as claimed in any preceding claim, wherein the one or more biochemical and/or biophysical parameters of the microorganisms is determined by assessing the uptake of one of more fluorescent or other stains.

4. The method as claimed in any preceding claim, wherein one or more biochemical and/or biophysical parameters of the microorganisms in both mixtures are assessed.

5. The method as claimed in any preceding claim, wherein the carbocyanine dye is present in the mixtures at a concentrate in the range of 1 $\mu$M to 5 $\mu$M, and optionally, 3 $\mu$M.

6. The method as claimed in any preceding claim, wherein the flow cytometer relies upon excitation at 640 nm and the parameters are assessed at 675$\pm$15 nm.

7. The method as claimed in claim 6, wherein the parameter comprises nucleic acid content and/or distribution and DNA distribution is assessed on the flow cytometer at 572 nm.

8. The method as claimed in claim 7, wherein the nucleic acid strain comprises SYBR Green.

9. The method as claimed in any preceding claim, wherein the growth medium comprises Terrific Broth.

10. The method as claimed in any preceding claim, wherein step c) takes place at a temperature in the range of 35°C and 40°C, and optionally, at a temperature of 37°C.

11. The method as claim in any preceding claim, wherein a portion of the first and second mixture, or portion of the one or more further mixtures when dependent upon claim 2, is assessed at multiple time points, and optionally, wherein the multiple time points comprise one or more of the following time points, 0 minutes, 5 minutes, 10 minutes, 15 minutes and/or less than 20 minutes.

12. The method as claimed in any preceding claim, wherein the microorganism is obtained from a biological sample derived from an individual believed to be suffering from a microorganism infection, and optionally, wherein the microorganism infection is a Urinary Tract Infection (UTI).

13. The method as claimed in any preceding claim, for determining the antimicrobial agent for use in the treatment of a microorganism infection in an individual, wherein the method comprises taking a biological sample from the individual, assessing the susceptibility of the microorganism, in the biological sample, to two or more antimicrobial agents and identifying which antimicrobial agent to administer to the individual based which antimicrobial agent inhibits or slows the proliferation of the microorganism.

**Patentansprüche**

1. Verfahren zur raschen Bestimmung der Suszeptibilität eines Mikroorganismus in einer Urinprobe gegenüber einem antimikrobiellen Mittel, umfassend die folgenden Schritte:

a) Inkontaktbringen einer ersten Probe, die den Mikroorganismus enthält, mit einem ersten Wachstumsmedium, um ein erstes Gemisch zu bilden, wobei das erste Wachstumsmedium so ausgewählt ist, dass es dem Mikroorganismus die Proliferation ermöglicht und/oder den Zellzyklus des Mikroorganismus zum Beginnen der Proliferation anregt;

b) Inkontaktbringen einer zweiten Probe, die den Mikroorganismus enthält, mit einem zweiten Wachstumsmedium, um ein zweites Gemisch zu bilden, wobei das zweite Wachstumsmedium im Wesentlichen identisch mit dem ersten Wachstumsmedium ist, aber ferner ein erstes antimikrobielles Mittel umfasst, das die Proliferation des Mikroorganismus hemmen oder verlangsamen kann;

c) Inkubieren des ersten und des zweiten Gemischs für 30 Minuten oder weniger unter Bedingungen, die geeignet sind, die Proliferation des Mikroorganismus zu ermöglichen oder anzuregen;

d) Auswerten des ersten und des zweiten Gemischs oder eines Teils davon mithilfe eines Durchflusszytometers, um einen oder mehrere biochemische und/oder biophysikalische Parameter der Mikroorganismen in beiden Gemischen auszuwerten; und

e) Vergleichen der Parameter der Mikroorganismen im ersten Gemisch mit denen des zweiten Gemischs nach der Inkubation, um nachzuweisen, ob das erste antimikrobielle Mittel die Proliferation des Mikroorganismus hemmt oder verlangsamt, um so die Suszeptibilität eines Mikroorganismus gegenüber dem Mittel zu bestimmen,

wobei es sich bei den Parametern handelt um:

(i) Zellgröße, Zellzahl und/oder Zellmembran-Energetisierung, und wobei vor dem Schritt d) Carbocyanin-Farbstoff, umfassend 3,3'-Dipropylthiadicarbocyaniniodid (di-S-C3(5)), dem Gemisch oder einem Teil des Gemischs zugesetzt wird; und

(ii) Nukleinsäuregehalt und/oder -verteilung, und wobei die Nukleinsäure DNA umfasst und wobei vor dem Schritt d) Mithramycin und ein Nukleinsäure-Färbemittel, umfassend SYBR-Green oder Ethidiumbromid, dem Gemisch oder einem Teil des Gemischs zugesetzt werden.

2. Verfahren nach Anspruch 1, wobei Schritt b) ferner das Inkontaktbringen einer oder mehrerer weiterer Proben, die den Mikroorganismus enthalten, mit einem oder mehreren weiteren Wachstumsmedien umfasst, um ein oder mehrere weitere Gemische zu bilden, wobei das eine oder die mehreren weiteren Wachstumsmedien identisch mit dem ersten Wachstumsmedium ist/sind, aber ferner ein oder mehrere weitere antimikrobielle Mittel umfasst/umfassen, welche(s) die Proliferation des Mikroorganismus hemmen oder verlangsamen kann/können, wobei das eine oder die mehreren weiteren antimikrobiellen Mittel voneinander verschieden und vom ersten antimikrobiellen Mittel verschieden sind.

3. Verfahren nach einem der vorangehenden Ansprüche, wobei der eine oder die mehreren biochemischen und/oder biophysikalischen Parameter der Mikroorganismen durch Auswerten der Aufnahme von einem oder mehreren fluoreszenten oder anderen Färbemitteln bestimmt wird/werden.

4. Verfahren nach einem der vorangehenden Ansprüche, wobei ein oder mehrere biochemische und/oder biophysikalische Parameter der Mikroorganismen in beiden Gemischen ausgewertet werden.

5. Verfahren nach einem der vorangehenden Ansprüche, wobei der Carbocyanin-Farbstoff in den Gemischen bei einer Konzentration im Bereich von 1 μM bis 5 μM, und optional bei 3 μM, vorhanden ist.

6. Verfahren nach einem der vorangehenden Ansprüche, wobei das Durchflusszytometer auf Erregung bei 640 nm beruht, und die Parameter bei $675 \pm 15$ nm ausgewertet werden.

7. Verfahren nach Anspruch 6, wobei der Parameter Nukleinsäuregehalt und/oder -verteilung umfasst, und die DNA-Verteilung auf dem Durchflusszytometer bei 572 nm ausgewertet wird.

8. Verfahren nach Anspruch 7, wobei das Nukleinsäure-Färbemittel SYBR-Green umfasst.

9. Verfahren nach einem der vorangehenden Ansprüche, wobei das Wachstumsmedium Terrific Broth umfasst.

10. Verfahren nach einem der vorangehenden Ansprüche, wobei Schritt c) bei einer Temperatur im Bereich von 35°C und 40°C, und optional bei einer Temperatur von 37°C stattfindet.

11. Verfahren nach einem der vorangehenden Ansprüche, wobei ein Teil des ersten und des zweiten Gemischs, oder ein Teil des einen oder der mehreren weiteren Gemische, wenn abhängig von Anspruch 2, zu mehreren Zeitpunkten ausgewertet wird, und wobei optional die mehreren Zeitpunkte einen oder mehrere der folgenden Zeitpunkte umfassen: 0 Minuten, 5 Minuten, 10 Minuten, 15 Minuten und/oder weniger als 20 Minuten.

**12.** Verfahren nach einem der vorangehenden Ansprüche, wobei der Mikroorganismus aus einer biologischen Probe gewonnen wird, die von einem Individuum stammt, von dem angenommen wird, dass es an einer Mikroorganismus-Infektion leidet, und wobei es sich bei der Mikroorganismus-Infektion optional um eine Harnwegsinfektion (UTI, Urinary Tract Infection) handelt.

**13.** Verfahren nach einem der vorangehenden Ansprüche zur Bestimmung des antimikrobiellen Mittels zur Verwendung bei der Behandlung einer Mikroorganismus-Infektion bei einem Individuum, wobei das Verfahren die Entnahme einer biologischen Probe von dem Individuum, die Auswertung der Suszeptibilität des Mikroorganismus in der biologischen Probe gegenüber zwei oder mehreren antimikrobiellen Mitteln und die Identifizierung, welches antimikrobielle Mittel dem Individuum verabreicht werden soll, basierend darauf, welches antimikrobielle Mittel die Proliferation des Mikroorganismus hemmt oder verlangsamt, umfasst.

## Revendications

**1.** Procédé pour déterminer rapidement la susceptibilité d'un microorganisme dans un échantillon d'urine à un agent antimicrobien comprenant les étapes de :

a) mise en contact d'un premier échantillon contenant le microorganisme avec un premier milieu de culture de façon à former un premier mélange, dans lequel le premier milieu de culture est sélectionné de façon à permettre au microorganisme de proliférer et/ou stimuler le cycle cellulaire du microorganisme pour commencer la prolifération ;
b) mise en contact d'un deuxième échantillon contenant le microorganisme avec un deuxième milieu de culture de façon à former un deuxième mélange, dans lequel le deuxième milieu de culture est sensiblement identique au premier milieu de culture mais comprend en outre un premier agent antimicrobien qui peut inhiber ou ralentir la prolifération du microorganisme ;
c) incubation des premier et deuxième mélanges, pendant 30 minutes ou moins, dans des conditions adaptées pour permettre ou favoriser la prolifération du microorganisme ;
d) évaluation des premier et deuxième mélanges, ou une partie de ceux-ci, au moyen d'un cytomètre en flux afin d'évaluer un ou plusieurs paramètres biochimiques et/ou biophysiques des microorganismes dans les deux mélanges ; et
e) comparaison des paramètres des microorganismes dans le premier mélange à ceux du deuxième mélange, après incubation, afin de détecter si le premier agent antimicrobien inhibe ou ralentit la prolifération du microorganisme de façon à déterminer la susceptibilité d'un microorganisme audit agent,

dans lequel les paramètres sont :

(i) la taille de cellule, le nombre de cellules et/ou l'énergisation de la membrane cellulaire, et dans lequel, avant l'étape d), un colorant carbocyanine, comprenant l'iodure de 3,3'-dipropylthiadicarbocyanine (di-S-C3(5)), est ajouté au mélange ou à une partie du mélange ; et
(ii) la teneur et/ou la distribution d'acide nucléique et ledit acide nucléique comprend de l'ADN et dans lequel, avant l'étape d), de la mithramycine et un colorant d'acide nucléique, comprenant SYBR Green ou le bromure d'éthidium, sont ajoutés au mélange ou à une partie du mélange.

**2.** Procédé selon la revendication 1, dans lequel l'étape b) comprend en outre la mise en contact d'un ou plusieurs échantillons supplémentaires contenant le microorganisme avec un ou plusieurs milieux de culture supplémentaires de façon à former un ou plusieurs mélanges supplémentaires, dans lequel les un ou plusieurs milieux de culture supplémentaires sont les mêmes que le premier milieu de culture mais comprennent en outre un ou plusieurs agents antimicrobiens supplémentaires qui peuvent inhiber ou ralentir la prolifération du microorganisme, dans lequel lesdits un ou plusieurs agents antimicrobiens supplémentaires sont différents les uns des autres et différents du premier agent antimicrobien.

**3.** Procédé selon l'une quelconque des revendications précédentes, dans lequel les un ou plusieurs paramètres biochimiques et/ou biophysiques des microorganismes sont déterminés par évaluation de l'absorption d'un ou plusieurs colorants fluorescents ou autres.

**4.** Procédé selon l'une quelconque des revendications précédentes, dans lequel un ou plusieurs paramètres biochimiques et/ou biophysiques des microorganismes dans les deux mélanges sont évalués.

5. Procédé selon l'une quelconque des revendications précédentes, dans lequel le colorant carbocyanine est présent dans les mélanges à une concentration dans la plage de 1 μM à 5 μM, et facultativement, 3 μM.

6. Procédé selon l'une quelconque des revendications précédentes, dans lequel le cytomètre en flux est basé sur une excitation à 640 nm et les paramètres sont évalués à 675 ± 15 nm.

7. Procédé selon la revendication 6, dans lequel le paramètre comprend une teneur et/ou distribution d'acide nucléique et la distribution d'ADN est évaluée sur le cytomètre en flux à 572 nm.

8. Procédé selon la revendication 7, dans lequel le colorant d'acide nucléique comprend SYBR Green.

9. Procédé selon l'une quelconque des revendications précédentes, dans lequel le milieu de culture comprend Terrific Broth.

10. Procédé selon l'une quelconque des revendications précédentes, dans lequel l'étape c) est effectuée à une température dans la plage de 35 °C à 40 °C, et facultativement, à une température de 37 °C.

11. Procédé selon l'une quelconque des revendications précédentes, dans lequel une partie des premier et deuxième mélanges, ou une partie des un ou plusieurs mélanges supplémentaires lorsqu'ils sont dépendants de la revendication 2, est évaluée à des temps multiples, et facultativement, dans lequel les temps multiples comprennent un ou plusieurs des temps suivants, 0 minute, 5 minutes, 10 minutes, 15 minutes et/ou moins de 20 minutes.

12. Procédé selon l'une quelconque des revendications précédentes, dans lequel le microorganisme est obtenu à partir d'un échantillon biologique dérivé d'un individu suspecté de souffrir d'une infection par un microorganisme, et facultativement, dans lequel l'infection par un microorganisme est une infection des voies urinaires (IVU).

13. Procédé selon l'une quelconque des revendications précédentes, pour déterminer l'agent antimicrobien pour utilisation dans le traitement d'une infection par un microorganisme chez un individu, le procédé comprenant le prélèvement d'un échantillon biologique à partir de l'individu, l'évaluation de la susceptibilité du microorganisme, dans l'échantillon biologique, à au moins deux agents antimicrobiens et l'identification de l'agent antimicrobien à administrer à l'individu sur la base de quel agent antimicrobien inhibe ou ralentit la prolifération du microorganisme.

Figure 1

EP 3 887 535 B1

**Figure 2**

**A**

| | | Sample name | Count |
|---|---|---|---|
| | | Replicate 1 | 989 |
| | | Replicate 2 | 938 |
| | | Replicate 3 | 985 |
| | | Replicate 4 | 918 |
| | | Replicate 5 | 983 |

$10^4$        $10^5$        $10^6$        $10^7$

**RL1 (red fluorescence)**

**Figure 2 cont.**

B

| | | Sample name | Count |
|---|---|---|---|
| | | Beads | 10884 |
| | | Replicate 1 | 989 |
| | | Replicate 2 | 938 |
| | | Replicate 3 | 985 |
| | | Replicate 4 | 918 |
| | | Replicate 5 | 983 |

$10^0$  $10^1$  $10^2$  $10^3$  $10^4$  $10^5$  $10^6$  $10^7$

**RL1 (red fluorescence)**

**Figure 2 cont.**

C

D

**Figure 2 cont.**

**Figure 2E cont.**

**Figure 2E cont.**

Figure 3

A

| | Incubation time | Count |
|---|---|---|
| | 0 minutes | 293 |
| | 5 minutes | 300 |
| | 10 minutes | 317 |
| | 15 minutes | 352 |
| | 20 minutes | 436 |
| | 25 minutes | 496 |
| | 30 minutes | 646 |

**Figure 3 cont.**

**B** $10^5$ **cells.mL$^{-1}$**

| Incubation Time | Negative control (TB with DiSC3) | 0 min (control) | 5 mins | 10 mins | 15 mins | 20 mins | 25 mins | 30 mins |
|---|---|---|---|---|---|---|---|---|
| Replicate 1 | 17 | 301 | 299 | 290 | 359 | 450 | 477 | 636 |
| Replicate 2 | 29 | 279 | 291 | 324 | 354 | 423 | 498 | 630 |
| Replicate 3 | 14 | 284 | 317 | 320 | 336 | 443 | 495 | 657 |
| Replicate 4 | 16 | 293 | 305 | 326 | 351 | 435 | 502 | 646 |
| Replicate 5 | 17 | 308 | 287 | 325 | 360 | 427 | 510 | 661 |
| Average | 19 | 293 | 300 | 317 | 352 | 436 | 496 | 646 |
| Standard deviation | 5.31 | 10.54 | 10.63 | 13.80 | 8.65 | 9.93 | 10.93 | 11.91 |
| Z' statistics (w.r.t. control) | | | | -2.07 | 0.02 | 0.57 | 0.90 | 0.81 |

**C** **5.104 cells.mL-1**

| Incubation Time | 0 min (control) | 10 mins | 20 mins | 25 mins | 30 mins |
|---|---|---|---|---|---|
| Replicate 1 | 111 | 180 | 228 | 265 | 363 |
| Replicate 2 | 111 | 192 | 200 | 240 | 428 |
| Replicate 3 | 91 | 183 | 210 | 252 | 355 |
| Replicate 4 | 93 | 166 | 195 | 255 | 363 |
| Replicate 5 | 118 | 177 | 201 | 255 | 374 |
| Average | 105 | 180 | 207 | 253 | 377 |
| Standard deviation | 10.78 | 8.45 | 11.65 | 8.01 | 26.40 |
| Z' statistics (w.r.t. control) | | 0.34 | 0.62 | 0.59 | |

## Figure 4

**A**

| Incubation time | Count |
|---|---|
| 0 minutes | 493 |
| 5 minutes | 538 |
| 10 minutes | 646 |
| 15 minutes | 684 |
| 20 minutes | 737 |
| 25 minutes | 779 |
| 30 minutes | 875 |

0 minutes
5 minutes
10 minutes
15 minutes
20 minutes
25 minutes
30 minutes

$10^4$    $10^5$    $10^6$

**RL1-H**

**B**

| Incubation time | Count |
|---|---|
| 0 minutes | 489 |
| 5 minutes | 534 |
| 10 minutes | 597 |
| 15 minutes | 626 |
| 20 minutes | 685 |
| 25 minutes | 708 |
| 30 minutes | 762 |

0 minutes
5 minutes
10 minutes
15 minutes
20 minutes
25 minutes
30 minutes

$10^4$    $10^5$    $10^6$

**RL1-H**

**Figure 4 cont.**

C

| Incubation time | Count |
|---|---|
| 0 minutes | 507 |
| 5 minutes | 549 |
| 10 minutes | 644 |
| 15 minutes | 650 |
| 20 minutes | 666 |
| 25 minutes | 707 |
| 30 minutes | 803 |

0 minutes
5 minutes
10 minutes
15 minutes
20 minutes
25 minutes
30 minutes

$10^4$     $10^5$     $10^6$

**RL1-H**

D

| Incubation time | Count |
|---|---|
| 0 minutes | 530 |
| 5 minutes | 615 |
| 10 minutes | 711 |
| 15 minutes | 685 |
| 20 minutes | 396 |
| 25 minutes | 338 |
| 30 minutes | 192 |

0 minutes
5 minutes
10 minutes
15 minutes
20 minutes
25 minutes
30

$10^4$     $10^5$     $10^6$

**RL1-H**

# Figure 4 cont.

**Strain 7 (sensitive cell line)**

| Incubation Time | 0 mins w/o Amp | 0 mins w/ Amp | 5 mins w/o Amp | 5 mins w/ Amp | 10 mins w/o Amp | 10 mins w/ Amp | 15 mins w/o Amp | 15 mins w/ Amp | 20 mins w/o Amp | 20 mins w/ Amp | 25 mins w/o Amp | 25 mins w/ Amp | 30 mins w/o Amp | 30 mins w/ Amp |
|---|---|---|---|---|---|---|---|---|---|---|---|---|---|---|
| Replicate 1 | 476 | 510 | 534 | 616 | 671 | 725 | 648 | 663 | 702 | 374 | 667 | 358 | 814 | 177 |
| Replicate 2 | 503 | 539 | 573 | 629 | 627 | 729 | 641 | 704 | 669 | 397 | 734 | 338 | 834 | 198 |
| Replicate 3 | 534 | 556 | 541 | 601 | 663 | 684 | 682 | 699 | 621 | 432 | 719 | 341 | 765 | 196 |
| Replicate 4 | 515 | 515 | 546 | 614 | 614 | 705 | 630 | 674 | 673 | 382 | 709 | 313 | 798 | 198 |
| Average | 507 | 530 | 549 | 615 | 644 | 711 | 650 | 685 | 646 | 396 | 707 | 338 | 803 | 192 |
| Std Dev (%) | 21.22 | 18.59 | 14.74 | 9.92 | 23.87 | 17.92 | 19.42 | 17.04 | 29.22 | 22.11 | 24.88 | 16.07 | 25.44 | 8.71 |
| Cell count difference (wrt control) | | | 42 | 45 | 137 | 181 | 143 | 155 | 139 | 134 | 200 | 100 | 296 | 135 |

**Strain 16 (resistant cell line)**

| Incubation Time | 0 mins w/o Amp | 0 mins w/ Amp | 5 mins w/o Amp | 5 mins w/ Amp | 10 mins w/o Amp | 10 mins w/ Amp | 15 mins w/o Amp | 15 mins w/ Amp | 20 mins w/o Amp | 20 mins w/ Amp | 25 mins w/o Amp | 25 mins w/ Amp | 30 mins w/o Amp | 30 mins w/ Amp |
|---|---|---|---|---|---|---|---|---|---|---|---|---|---|---|
| Replicate 1 | 509 | 454 | 516 | 534 | 656 | 575 | 706 | 635 | 754 | 705 | 769 | 681 | 763 | 832 |
| Replicate 2 | 486 | 480 | 538 | 516 | 694 | 588 | 645 | 612 | 731 | 704 | 787 | 725 | 743 | 904 |
| Replicate 3 | 484 | 515 | 550 | 549 | 657 | 616 | 705 | 623 | 739 | 646 | 762 | 709 | 779 | 842 |
| Replicate 4 | 493 | 507 | 547 | 537 | 577 | 610 | 679 | 633 | 723 | 685 | 799 | 715 | 764 | 862 |
| Average | 493 | 489 | 538 | 534 | 646 | 597 | 684 | 626 | 737 | 685 | 779 | 708 | 762 | 875 |
| Std Dev (%) | 10.01 | 24.16 | 13.31 | 11.81 | 42.64 | 16.67 | 24.85 | 9.05 | 11.56 | 23.76 | 14.60 | 16.33 | 12.73 | 24.22 |
| Cell count difference (wrt control) | | | 45 | 45 | 153 | 108 | 191 | 137 | 244 | 196 | 286 | 218 | 382 | 273 |

E

**Figure 5A**

| Incubation time | Count | $10^3$. Mode : SSC |
|---|---|---|
| 0 minutes | 493 | 21.2 |
| 5 minutes | 538 | 26.4 |
| 10 minutes | 646 | 23.7 |
| 15 minutes | 684 | 25.9 |
| 20 minutes | 737 | 22.4 |
| 25 minutes | 779 | 28.3 |
| 30 minutes | 875 | 26.4 |

0 minutes

5 minutes

10 minutes

15 minutes

20 minutes

25 minutes

30 minutes

$10^3$       $10^4$       $10^5$

**SSC-H**

**Figure 5B**

| Incubation time | Count | $10^3$. Mode : SSC |
|---|---|---|
| 0 minutes | 489 | 20.9 |
| 5 minutes | 534 | 23.7 |
| 10 minutes | 597 | 22.8 |
| 15 minutes | 626 | 24.1 |
| 20 minutes | 685 | 24.1 |
| 25 minutes | 708 | 26.8 |
| 30 minutes | 762 | 25.9 |

**Figure 5C**

| Incubation time | Count | $10^3$. Mode : SSC |
|---|---|---|
| 0 minutes | 507 | 28.3 |
| 5 minutes | 549 | 26.8 |
| 10 minutes | 644 | 29.9 |
| 15 minutes | 650 | 37.1 |
| 20 minutes | 666 | 33.3 |
| 25 minutes | 707 | 35.8 |
| 30 minutes | 803 | 35.8 |

**Figure 5D**

| Incubation time | Count | 10³ . Mode : SSC |
|---|---|---|
| 0 minutes | 530 | 22.8 |
| 5 minutes | 615 | 29.9 |
| 10 minutes | 711 | 26.8 |
| 15 minutes | 685 | 39.9 |
| 20 minutes | 396 | 35.8 |
| 25 minutes | 338 | 35.8 |
| 30 minutes | 192 | 35.8 |

Figure 6A

Figure 6B

Figure 7

| | Antibiotic sensitivity (R- resistant; S- sensitive) | | | |
|---|---|---|---|---|
| Sample Date | Ampicillin | Trimethoprim | Ciprofloxacin | Nitrofurantoin |
| 25/05/2018 | R | R | S | S |
| 25/05/2018 | S | S | S | S |
| 06/06/2018 | R | S | S | S |
| 16/07/2018 | R | S | S | S |
| 18/07/2018 | R | S | R | R |
| 20/07/2018 | S | R | S | S |

**Figure 8A**

**Figure 8B**

| Incubation time | Count |
|:---:|:---:|
| 0 minutes | 261 |
| 5 minutes | 241 |
| 10 minutes | 304 |
| 15 minutes | 281 |
| 20 minutes | 221 |
| 30 minutes | 121 |

**Figure 8C**

| Incubation time | Count |
|---|---|
| 0 minutes | 346 |
| 5 minutes | 338 |
| 10 minutes | 253 |
| 15 minutes | 390 |
| 20 minutes | 655 |
| 30 minutes | 726 |

**Figure 8D**

| Incubation time | Count |
|:---:|:---:|
| 0 minutes | 346 |
| 5 minutes | 338 |
| 10 minutes | 253 |
| 15 minutes | 390 |
| 20 minutes | 655 |
| 30 minutes | 726 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2018184073 A
- WO 2010129532 A
- US 2018195964 A
- US 2003103936 A

### Non-patent literature cited in the description

- **J V ORDONEZ et al.** *Cytometry,* 1993, vol. 14 (7), 811-818
- **MASON A D J et al.** *J. Appl. Bacteriol.,* 1995, vol. 78 (3), 309-315
- **V S FORSYTH et al.** *American Societ. Biol.,* 2018, vol. 9 (2), e00186-181, 18, , 1-8
- **YANG S T et al.** *Biochemica et Biophysica Acta,* 2006, vol. 1758 (10), 1580-1586
- **AGUIRRE JS ; GONZALEZ A ; OZCELIK N ; ROD-RIGUEZ MR ; GARCIA DE FERNANDO GD.** Modeling the Listeria innocua micropopulation lag phase and its variability. *Int J Food Microbiol,* 2013, vol. 164, 60-69
- **AGUIRRE JS ; KOUTSOUMANIS KP.** Towards lag phase of microbial populations at growth-limiting conditions: The role of the variability in the growth limits of individual cells. *Int J Food Microbiol,* 2016, vol. 224, 1-6
- **ÅKERLUND T ; NORDSTRÖM K ; BERNANDER R.** Analysis of cell size and DNA content in exponentially growing and stationary-phase batch cultures of Escherichia coli. *J Bacteriol,* 1995, vol. 177, 6791-6797
- **ÁLVAREZ-BARRIENTOS A ; ARROYO J ; CANTÓN R ; NOMBELA C ; SÁNCHEZ-PÉREZ M.** Applications of flow cytometry to clinical microbiology. *Clin Microbiol Rev,* 2000, vol. 13, 167-195
- **ANDERSSON DI ; HUGHES D.** Antibiotic resistance and its cost: is it possible to reverse resistance?. *Nat Rev Microbiol,* 2010, vol. 8, 260-271
- **BAKER S ; THOMSON N ; WEILL FX ; HOLT KE.** Genomic insights into the emergence and spread of antimicrobial-resistant bacterial pathogens. *Science,* 2018, vol. 360, 733-738
- **BALTEKIN Ö ; BOUCHARIN A ; TANO E ; ANDERSSON DI ; ELF J.** Antibiotic susceptibility testing in less than 30 min using direct single-cell imaging. *Proc Natl Acad Sci U S A,* 2017, vol. 114, 9170-9175
- **BARANYI J ; PIN C.** Estimating bacterial growth parameters by means of detection times. *Appl Environ Microbiol,* 1999, vol. 65, 732-736
- **BARANYI J ; ROBERTS TA.** A dynamic approach to predicting bacterial growth in food. *Int J Food Microbiol,* 1994, vol. 23, 277-294
- **BASHFORD CL.** The measurement of membrane potential using optical indicators. *Biosci Rep,* 1981, vol. 1, 183-196
- **BATY F ; DELIGNETTE-MULLER ML.** Estimating the bacterial lag time: which model, which precision?. *Int J Food Microbiol,* 2004, vol. 91, 261-277
- **BATY F ; FLANDROIS JP ; DELIGNETTE-MULLER ML.** Modeling the lag time of Listeria monocytogenes from viable count enumeration and optical density data. *Appl Environ Microbiol,* 2002, vol. 68, 5816-5825
- **BERTRAND RL.** Lag phase-associated iron accumulation is likely a microbial counter-strategy to host iron sequestration: role of the ferric uptake regulator (fur). *J Theor Biol,* 2014, vol. 359, 72-79
- **BOI P ; MANTI A ; PIANETTI A ; SABATINI L ; SISTI D ; ROCCHI MB ; BRUSCOLINI F ; GALLUZZI L ; PAPA S.** Evaluation of Escherichia coli viability by flow cytometry: A method for determining bacterial responses to antibiotic exposure. *Cytometry B Clin Cytom,* 2015, vol. 88, 149-153
- **BOYE E ; LØBNER-OLESEN A.** Bacterial Growth Control Studied by Flow Cytometry. *Res Microbiol,* 1991, vol. 142, 131-135
- **BOYE E ; STEEN HB ; SKARSTAD K.** Flow Cytometry of Bacteria: A Promising Tool in Experimental and Clinical Microbiology. *J Gen Microbiol,* 1983, vol. 129, 973-980
- **BROCHADO AR ; TELZEROW A ; BOBONIS J ; BANZHAF M ; MATEUS A ; SELKRIG J ; HUTH E ; BASSLER S ; ZAMARRENO BEAS J ; ZIETEK M.** Species-specific activity of antibacterial drug combinations. *Nature,* 2018, vol. 559, 259-263
- **BRYCE A ; HAY AD ; LANE IF ; THORNTON HV ; WOOTTON M ; COSTELLOE C.** Global prevalence of antibiotic resistance in paediatric urinary tract infections caused by Escherichia coli and association with routine use of antibiotics in primary care: systematic review and meta-analysis. *BMJ,* 2016, vol. 352, i939

- **BUCHAN BW ; LEDEBOER NA.** Emerging technologies for the clinical microbiology laboratory. *Clin Microbiol Rev,* 2014, vol. 27, 783-822
- **CEK M ; TANDOGDU Z ; WAGENLEHNER F ; TENKE P ; NABER K ; BJERKLUND-JOHANSEN TE.** Healthcare-associated urinary tract infections in hospitalized urological patients--a global perspective: results from the GPIU studies 2003-2010. *World J Urol,* 2014, vol. 32, 1587-1594
- **CHANDRA A ; SINGH N.** Bacterial growth sensing in microgels using pH-dependent fluorescence emission. *Chem Commun (Camb),* 2018, vol. 54, 1643-1646
- **CHIEN TL ; KAO JT ; LIU HL ; LIN PC ; HONG JS ; HSIEH HP ; CHIEN MJ.** Urine sediment examination: a comparison of automated urinalysis systems and manual microscopy. *Clin Chim Acta,* 2007, vol. 384, 28-34
- **CHOI J ; YOO J ; LEE M ; KIM EG ; LEE JS ; LEE S ; JOO S ; SONG SH ; KIM EC ; LEE JC.** A rapid antimicrobial susceptibility test based on single-cell morphological analysis. *Sci Transl Med,* 2014, vol. 6, 267-174
- **COATES AR ; HALLS G ; HU Y.** Novel classes of antibiotics or more of the same?. *Br J Pharmacol,* 2011, vol. 163, 184-194
- **COOPER S ; HELMSTETTER CE.** Chromosome Replication and the Division Cycle of Escherichia coli B/r. *J Mol Biol,* 1968, vol. 31, 519-540
- **DALGAARD P ; ROSS T ; KAMPERMAN L ; NEUMEYER K ; MCMEEKIN TA.** Estimation of bacterial growth rates from turbidimetric and viable count data. *Int J Food Microbiol,* 1994, vol. 23, 391-404
- **DAVEY HM.** Life, death, and in-between: meanings and methods in microbiology. *Appl Environ Microbiol,* 2011, vol. 77, 5571-5576
- **DAVEY HM ; KELL DB.** Flow cytometry and cell sorting of heterogeneous microbial populations: the importance of single-cell analysis. *Microbiol Rev,* 1996, vol. 60, 641-696
- **DETWEILER K ; MAYERS D ; FLETCHER SG.** Bacteruria and urinary tract infections in the elderly. *Urol Clin North Am,* 2015, vol. 42, 561-568
- **DIDELOT X ; BOWDEN R ; WILSON DJ ; PETO TEA ; CROOK DW.** Transforming clinical microbiology with bacterial genome sequencing. *Nat Rev Genet,* 2012, vol. 13, 601-612
- **DU D ; WANG-KAN X ; NEUBERGER A ; VAN VEEN HW ; POS KM ; PIDDOCK LJV ; LUISI BF.** Multidrug efflux pumps: structure, function and regulation. *Nat Rev Microbiol,* 2018
- **DUNNE WM, JR. ; JAILLARD M ; ROCHAS O ; VAN BELKUM A.** Microbial genomics and antimicrobial susceptibility testing. *Expert Rev Mol Diagn,* 2017, vol. 17, 257-269
- **EDWARDS BS ; YOUNG SM ; IVNITSKY-STEELE I ; YE RD ; PROSSNITZ ER ; SKLAR LA.** High-content screening: flow cytometry analysis. *Methods Mol Biol,* 2009, vol. 486, 151-165
- **EJRNAES K.** Bacterial characteristics of importance for recurrent urinary tract infections caused by Escherichia coli. *Dan Med Bull,* 2011, vol. 58, B4187
- **FARHA MA ; BROWN ED.** Chemical probes of Escherichia coli uncovered through chemical-chemical interaction profiling with compounds of known biological activity. *Chem Biol,* 2010, vol. 17, 852-862
- **FELLE H ; STETSON DL ; LONG WS ; SLAYMAN CL.** Direct measurement of membrane potential and resistance in giant cells of Escherichia colil. *Front Biol Energet,* 1978, vol. 2, 1399-1407
- **FINKEL SE.** Long-term survival during stationary phase: evolution and the GASP phenotype. *Nat Rev Microbiol,* 2006, vol. 4, 113-120
- **FOXMAN B.** The epidemiology of urinary tract infection. *Nat Rev Urol,* 2010, vol. 7, 653-660
- **GANT VA ; WARNES G ; PHILLIPS I ; SAVIDGE GF.** The application of flow cytometry to the study of bacterial responses to antibiotics. *J Med Microbiol,* 1993, vol. 39, 147-154
- **GELBAND H ; LAXMINARAYAN R.** Tackling antimicrobial resistance at global and local scales. *Trends Microbiol,* 2015, vol. 23, 524-526
- **GHAZI A ; SCHECHTER E ; LETELLIER L ; LABEDAN B.** Probes of membrane potential in Escherichia coli cells. *FEBS Lett,* 1981, vol. 125, 197-200
- **HAMMES F ; EGLI T.** Cytometric methods for measuring bacteria in water: advantages, pitfalls and applications. *Anal Bioanal Chem,* 2010, vol. 397, 1083-1095
- **HEWITT CJ ; NEBE-VON-CARON G.** The application of multi-parameter flow cytometry to monitor individual microbial cell physiological state. *Adv Biochem Eng Biotechnol,* 2004, vol. 89, 197-223
- **HIMEOKA Y ; KANEKO K.** Theory for Transitions Between Exponential and Stationary Phases: Universal Laws for Lag Time. *Phys Rev X,* 2017, vol. 7
- **HONG W ; KARANJA CW ; ABUTALEB NS ; YOUNIS W ; ZHANG X ; SELEEM MN ; CHENG JX.** Antibiotic susceptibility determination within one cell cycle at single-bacterium level by stimulated Raman metabolic imaging. *Anal Chem,* 2018, vol. 90, 3737-3743
- **IYER R ; FERRARI A ; RIJNBRAND R ; ERWIN AL.** A fluorescent microplate assay quantifies bacterial efflux and demonstrates two distinct compound binding sites in AcrB. *Antimicrob Agents Chemother,* 2015, vol. 59, 2388-2397
- **JERNAES MW ; STEEN HB.** Staining of Escherichia coli for flow cytometry: influx and efflux of ethidium bromide. *Cytometry,* 1994, vol. 17, 302-309

- **JÕERS A ; TENSON T.** Growth resumption from stationary phase reveals memory in Escherichia coli cultures. *Sci Rep,* 2016, vol. 6, 24055
- **JOHNSON LV ; WALSH ML ; BOCKUS BJ ; CHEN LB.** Monitoring of relative mitochondrial membrane potential in living cells by fluorescence microscopy. *J Cell Biol,* 1981, vol. 88, 526-535
- **KAPRELYANTS AS ; KELL DB.** Dormancy in stationary-phase cultures of Micrococcus luteus: flow cytometric analysis of starvation and resuscitation. *Appl Env Microbiol,* 1993, vol. 59, 3187-3196
- **KAPRELYANTS AS ; KELL DB.** Rapid assessment of bacterial viability and vitality using rhodamine 123 and flow cytometry. *J Appl Bacteriol,* 1992, vol. 72, 410-422
- **KELL DB ; DOBSON PD ; BILSLAND E ; OLIVER SG.** The promiscuous binding of pharmaceutical drugs and their transporter-mediated uptake into cells: what we (need to) know and how we can do so. *Drug Disc Today,* 2013, vol. 18, 218-239
- **KELL DB ; KAPRELYANTS AS ; WEICHART DH ; HARWOOD CL ; BARER MR.** Viability and activity in readily culturable bacteria: a review and discussion of the practical issues. *Antonie van Leeuwenhoek,* 1998, vol. 73, 169-187
- **KELL DB ; OLIVER SG.** How drugs get into cells: tested and testable predictions to help discriminate between transporter-mediated uptake and lipoidal bilayer diffusion. *Front Pharmacol,* 2014, vol. 5, 231
- **KELL DB ; POTGIETER M ; PRETORIUS E.** Individuality, phenotypic differentiation, dormancy and 'persistence' in culturable bacterial systems: commonalities shared by environmental, laboratory, and clinical microbiology. *F1000Res,* 2015, vol. 4, 179
- **KELL DB ; RYDER HM ; KAPRELYANTS AS ; WESTERHOFF HV.** Quantifying heterogeneity: Flow cytometry of bacterial cultures. *Antonie van Leeuwenhoek,* 1991, vol. 60, 145-158
- **KELLEY SO.** New technologies for rapid bacterial identification and antibiotic resistance profiling. *SLAS Technol,* 2017, vol. 22, 113-121
- **KERREMANS JJ ; VERBOOM P ; STIJNEN T ; HAKKAART-VAN ROIJEN L ; GOESSENS W ; VERBRUGH HA ; VOS MC.** Rapid identification and antimicrobial susceptibility testing reduce antibiotic use and accelerate pathogen-directed antibiotic use. *J Antimicrob Chemother,* 2008, vol. 61, 428-435
- **KESSEL D ; BECK WT ; KUKURUGA D ; SCHULZ V.** Characterization of multidrug resistance by fluorescent dyes. *Cancer Res,* 1991, vol. 51, 4665-4670
- **KIRCHHOFF J ; GLASER U ; BOHNERT JA ; PLETZ MW ; POPP J ; NEUGEBAUER U.** Simple Ciprofloxacin Resistance Test and Determination of Minimal Inhibitory Concentration within 2 h Using Raman Spectroscopy. *Anal Chem,* 2018, vol. 90, 1811-1818
- **KIRKUP BC ; MAHLEN S ; KALLSTROM G.** Future-generation sequencing and clinical microbiology. *Clinics in laboratory medicine,* 2013, vol. 33, 685-704
- **KLINE KA ; LEWIS AL.** Gram-Positive Uropathogens, Polymicrobial Urinary Tract Infection, and the Emerging Microbiota of the Urinary Tract. *Microbiology spectrum,* 2016, vol. 4
- **KOHANSKI MA ; DWYER DJ ; HAYETE B ; LAWRENCE CA ; COLLINS JJ.** A common mechanism of cellular death induced by bactericidal antibiotics. *Cell,* 2007, vol. 130, 797-810
- **KOKEN T ; AKTEPE OC ; SERTESER M ; SAMLI M ; KAHRAMAN A ; DOGAN N.** Determination of cutoff values for leucocytes and bacteria for urine flow cytometer (UF-100) in urinary tract infections. *Int Urol Nephrol,* 2002, vol. 34, 175-178
- **KÖSER CU ; ELLINGTON MJ ; CARTWRIGHT EJ ; GILLESPIE SH ; BROWN NM ; FARRINGTON M ; HOLDEN MT ; DOUGAN G ; BENTLEY SD ; PARKHILL J.** Routine use of microbial whole genome sequencing in diagnostic and public health microbiology. *PLoS pathogens,* 2012, vol. 8, e1002824
- **KÖVES B ; CAI T ; VEERATTERAPILLAY R ; PICKARD R ; SEISEN T ; LAM TB ; YUAN CY ; BRUYERE F ; WAGENLEHNER F ; BARTOLETTI R.** Benefits and Harms of Treatment of Asymptomatic Bacteriuria: A Systematic Review and Meta-analysis by the European Association of Urology Urological Infection Guidelines Panel. *Eur Urol,* 2017
- **KWONG JC ; MCCALLUM N ; SINTCHENKO V ; HOWDEN BP.** Whole genome sequencing in clinical and public health microbiology. *Pathology,* 2015, vol. 47, 199-210
- **LAXMINARAYAN R ; SRIDHAR D ; BLASER M ; WANG M ; WOOLHOUSE M.** Achieving global targets for antimicrobial resistance. *Science,* 2016, vol. 353, 874-875
- **LI B ; QIU Y ; SHI H ; YIN H.** The importance of lag time extension in determining bacterial resistance to antibiotics. *Analyst,* 2016, vol. 141, 3059-3067
- **LINK H ; FUHRER T ; GEROSA L ; ZAMBONI N ; SAUER U.** Real-time metabolome profiling of the metabolic switch between starvation and growth. *Nat Methods,* 2015, vol. 12, 1091-1097
- **MACEDO RS ; ONITA JH ; WILLE MP ; FURTADO GH.** Pharmacokinetics and pharmacodynamics of antimicrobial drugs in intensive care unit patients. *Shock,* 2013, vol. 39 (1), 24-28

- **MADAR D ; DEKEL E ; BREN A ; ZIMMER A ; PORAT Z ; ALON U.** Promoter activity dynamics in the lag phase of Escherichia coli. *BMC Syst Biol,* 2013, vol. 7, 136
- **MASON DJ ; ALLMAN R ; STARK JM ; LLOYD D.** Rapid Estimation of Bacterial Antibiotic Susceptibility With Flow- Cytometry. *Journal of Microscopy-Oxford,* 1994, vol. 176, 8-16
- **MASON DJ ; POWER EGM ; TALSANIA H ; PHILLIPS I ; GANT VA.** Antibacterial action of ciprofloxacin. *Antimicrob Agents Ch,* 1995, vol. 39, 2752-2758
- **MEHNERT-KAY SA.** Diagnosis and management of uncomplicated urinary tract infections. *American Family Physician,* 2005, vol. 72, 451-456
- **MENDELSON M ; BALASEGARAM M ; JINKS T ; PULCINI C ; SHARLAND M.** Antibiotic resistance has a language problem. *Nature,* 2017, vol. 545, 23-25
- **MODY L ; JUTHANI-MEHTA M.** Urinary tract infections in older women: a clinical review. *JAMA,* 2014, vol. 311, 844-854
- **MÜLLER S ; LOSCHE A ; BLEY T.** Staining procedures for flow cytometric monitoring of bacterial populations. *Acta Biotechnol,* 1993, vol. 13, 289-297
- **MÜLLER S ; NEBE-VON-CARON G.** Functional single-cell analyses: flow cytometry and cell sorting of microbial populations and communities. *FEMS Microbiol Rev,* 2010, vol. 34, 554-587
- **MURRAY C ; ADEYIGA O ; OWSLEY K ; DI CARLO D.** Research highlights: microfluidic analysis of antimicrobial susceptibility. *Lab Chip,* 2015, vol. 15, 1226-1229
- **NAVARRO LLORENS JM ; TORMO A ; MARTINEZ-GARCIA E.** Stationary phase in gram-negative bacteria. *FEMS Microbiol Rev,* 2010, vol. 34, 476-495
- **NEBE-VON CARON G ; BADLEY RA.** Viability assessment of bacteria in mixed populations using flow cytometry. *J Microsc,* 1995, vol. 179, 55-66
- **NOVOTNA J ; VOHRADSKY J ; BERNDT P ; GRAMAJO H ; LANGEN H ; LI XM ; MINAS W ; ORSARIA L ; ROEDER D ; THOMPSON CJ.** Proteomic studies of diauxic lag in the differentiating prokaryote Streptomyces coelicolor reveal a regulatory network of stress-induced proteins and central metabolic enzymes. *Mol Microbiol,* 2003, vol. 48, 1289-1303
- **PIERETTI B ; BRUNATI P ; PINI B ; COLZANI C ; CONGEDO P ; ROCCHI M ; TERRAMOCCI R.** Diagnosis of bacteriuria and leukocyturia by automated flow cytometry compared with urine culture. *Journal of clinical microbiology,* 2010, vol. 48, 3990-3996
- **PIN C ; BARANYI J.** Kinetics of single cells: observation and modeling of a stochastic process. *Appl Environ Microbiol,* 2006, vol. 72, 2163-2169
- **PIN C ; BARANYI J.** Single-cell and population lag times as a function of cell age. *Appl Environ Microbiol,* 2008, vol. 74, 2534-2536
- **PIN C ; ROLFE MD ; MUÑOZ-CUEVAS M ; HINTON JCD ; PECK MW ; WALTON NJ ; BARANYI J.** Network analysis of the transcriptional pattern of young and old cells of Escherichia coli during lag phase. *BMC Syst Biol,* 2009, vol. 3, 108
- **PIRT SJ.** Principles of microbe and cell cultivation. Wiley, 1975
- **PRATS C ; GIRÓ A ; FERRER J ; LÓPEZ D ; VIVES-REGO J.** Analysis and IbM simulation of the stages in bacterial lag phase: basis for an updated definition. *J Theor Biol,* 2008, vol. 252, 56-68
- **PRATS C ; LÓPEZ D ; GIRÓ A ; FERRER J ; VALLS J.** Individual-based modelling of bacterial cultures to study the microscopic causes of the lag phase. *J Theor Biol,* 2006, vol. 241, 939-953
- **ROACH DJ ; BURTON JN ; LEE C ; STACKHOUSE B ; BUTLER-WU SM ; COOKSON BT ; SHENDURE J ; SALIPANTE SJ.** A year of infection in the Intensive Care Unit: prospective whole genome sequencing of bacterial clinical isolates reveals cryptic transmissions and novel microbiota. *PLoS Genet,* 2015, vol. 11, e1005413
- **ROCA I ; AKOVA M ; BAQUERO F ; CARLET J ; CAVALERI M ; COENEN S ; COHEN J ; FINDLAY D ; GYSSENS I ; HEURE OE.** The global threat of antimicrobial resistance: science for intervention. *New Microbes New Infect,* 2015, vol. 6, 22-29
- **ROLFE MD ; RICE CJ ; LUCCHINI S ; PIN C ; THOMPSON A ; CAMERON ADS ; ALSTON M ; STRINGER MF ; BETTS RP ; BARANYI J.** Lag phase is a distinct growth phase that prepares bacteria for exponential growth and involves transient metal accumulation. *J Bacterio,* 2012, vol. 194, 686-701
- **ROOSTALU J ; JÕERS A ; LUIDALEPP H ; KALDALU N ; TENSON T.** Cell division in Escherichia coli cultures monitored at single cell resolution. *BMC Microbiol,* 2008, vol. 8
- **ROTTENBERG H.** The measurement of membrane potential and deltapH in cells, organelles, and vesicles. *Methods Enzymol,* 1979, vol. 55, 547-569
- **SAULS JT ; LI D ; JUN S.** Adder and a coarse-grained approach to cell size homeostasis in bacteria. *Curr Opin Cell Biol,* 2016, vol. 38, 38-44
- **SCHMIDT K ; MWAIGWISYA S ; CROSSMAN LC ; DOUMITH M ; MUNROE D ; PIRES C ; KHAN AM ; WOODFORD N ; SAUNDERS NJ ; WAIN J.** Identification of bacterial pathogens and antimicrobial resistance directly from clinical urines by nanopore-based metagenomic sequencing. *J Antimicr Chemother,* 2016

- **SCHMIEMANN G ; KNIEHL E ; GEBHARDT K ; MATEJCZYK MM ; HUMMERS-PRADIER E.** The diagnosis of urinary tract infection: a systematic review. *Dtsch Ärztebl Int,* 2010, vol. 107, 361-367
- **SCHOEPP NG ; SCHLAPPI TS ; CURTIS MS ; BUTKOVICH SS ; MILLER S ; HUMPHRIES RM ; ISMAGILOV RF.** Rapid pathogen-specific phenotypic antibiotic susceptibility testing using digital LAMP quantification in clinical samples. *Sci Transl Med,* 2017, vol. 9
- **SCHULTZ D ; KISHONY R.** Optimization and control in bacterial lag phase. *BMC Biol,* 2013, vol. 11, 120
- **SEAMER LC ; BAGWELL CB ; BARDEN L ; REDELMAN D ; SALZMAN GC ; WOOD JCS ; MURPHY RF.** Proposed new data file standard for flow cytometry, version FCS 3.0. *Cytometry,* 1997, vol. 28, 118-122
- **SENYUREK I ; PAULMANN M ; SINNBERG T ; KALBACHER H ; DEEG M ; GUTSMANN T ; HERMES M ; KOHLER T ; GOTZ F ; WOLZ C.** Dermcidin-derived peptides show a different mode of action than the cathelicidin LL-37 against Staphylococcus aureus. *Antimicrob Agents Chemother,* 2009, vol. 53, 2499-2509
- **SHANG YJ ; WANG QQ ; ZHANG JR ; XU YL ; ZHANG WW ; CHEN Y ; GU ML ; HU ZD ; DENG AM.** Systematic review and meta-analysis of flow cytometry in urinary tract infection screening. *Clin Chim Acta,* 2013, vol. 424, 90-95
- **SHAPIRO HM.** Flow cytometry of bacterial membrane potential and permeability. *Methods Mol Med,* 2008, vol. 142, 175-186
- **SHAPIRO HM.** Membrane potential estimation by flow cytometry. *Methods,* 2000, vol. 21, 271-279
- **SHAPIRO HM.** Multiparameter flow cytometry of bacteria: implications for diagnostics and therapeutics. *Cytometry,* 2001, vol. 43, 223-226
- **SHAPIRO HM.** Practical Flow Cytometry. John Wiley, 2003
- **SHAYANFAR N ; TOBLER U ; VON ECKARDSTEIN A ; BESTMANN L.** Automated urinalysis: first experiences and a comparison between the Iris iQ200 urine microscopy system, the Sysmex UF-100 flow cytometer and manual microscopic particle counting. *Clin Chem Lab Med,* 2007, vol. 45, 1251-1256
- **SI F ; LI D ; COX SE ; SAULS JT ; AZIZI O ; SOU C ; SCHWARTZ AB ; ERICKSTAD MJ ; JUN Y ; LI X.** Invariance of Initiation Mass and Predictability of Cell Size in Escherichia coli. *Curr Biol,* 2017, vol. 27, 1278-1287
- **SKARSTAD K ; BOYE E ; STEEN HB.** Timing of Initiation of Chromosome Replication in Individual Escherichia coli cells. *EMBO Journal,* 1986, vol. 5, 1711-1717
- **SKARSTAD K ; STEEN HB ; BOYE E.** Escherichia coli DNA Distributions Measured by Flow Cytometry and Compared with Theoretical Computer Simulations. *J Bacteriol,* 1985, vol. 163, 661-668
- **SKEGGS LT, JR.** An automatic method for colorimetric analysis. *Am J Clin Pathol,* 1957, vol. 28, 311-322
- **SKLAR LA ; CARTER MB ; EDWARDS BS.** Flow cytometry for drug discovery, receptor pharmacology and high-throughput screening. *Curr Opin Pharmacol,* 2007, vol. 7, 527-534
- Flow cytometric studies of microorganisms. **STEEN HB.** Flow Cytometry and Sorting. Wiley-Liss Inc, 1990, 605-622
- **STEEN HB ; BOYE E.** Bacterial growth studied by flow cytometry. *Cytometry,* 1980, vol. 1, 32-36
- **STOKKE C ; FLÅTTEN I ; SKARSTAD K.** An easy-to-use simulation program demonstrates variations in bacterial cell cycle parameters depending on medium and temperature. *PLoS One,* 2012, vol. 7, e30981
- **STYLIANIDOU S ; BRENNAN C ; NISSEN SB ; KUWADA NJ ; WIGGINS PA.** SuperSegger: robust image segmentation, analysis and lineage tracking of bacterial cells. *Mol Microbiol,* 2016, vol. 102, 690-700
- **SWINNEN IAM ; BERNAERTS K ; DENS EJJ ; GEERAERD AH ; VAN IMPE JF.** Predictive modelling of the microbial lag phase: a review. *Int J Food Microbiol,* 2004, vol. 94, 137-159
- **TAHERI-ARAGHI S ; BROWN SD ; SAULS JT ; MCLNTOSH DB ; JUN S.** Single-Cell Physiology. *Annu Rev Biophys,* 2015, vol. 44, 123-142
- **TANDOGDU Z ; WAGENLEHNER FM.** Global epidemiology of urinary tract infections. *Curr Opin Infect Dis,* 2016, vol. 29, 73-79
- **TARTOF KD ; HOBBS CA.** Improved Media for Growing Plasmid and Cosmid Clones. *Bethseda Research Laboratories Focus,* 1987, vol. 9, 12
- **TEGOS GP ; EVANGELISTI AM ; STROUSE JJ ; URSU O ; BOLOGA C ; SKLAR LA.** A high throughput flow cytometric assay platform targeting transporter inhibition. *Drug Disc Today Technol,* 2014, vol. 12, e95-e103
- **TSAI EA ; SHAKBATYAN R ; EVANS J ; ROSSETTI P ; GRAHAM C ; SHARMA H ; LIN CF ; LEBO MS.** Bioinformatics Workflow for Clinical Whole Genome Sequencing at Partners HealthCare Personalized Medicine. *J Pers Med,* 2016, vol. 6
- **TUITE N ; REDDINGTON K ; BARRY T ; ZUMLA A ; ENNE V.** Rapid nucleic acid diagnostics for the detection of antimicrobial resistance in Gram-negative bacteria: is it time for a paradigm shift?. *J Antimicrob Chemother,* 2014, vol. 69, 1729-1733
- **VAN BELKUM A ; DUNNE WM, JR.** Next-generation antimicrobial susceptibility testing. *Journal of clinical microbiology,* 2013, vol. 51, 2018-2024

- **WAGGONER A.** Optical probes of membrane potential. *J Membr Biol,* 1976, vol. 27, 317-334
- **WAGGONER AS.** Dye indicators of membrane potential. *Annu Rev Biophys Bioeng,* 1979, vol. 8, 47-68
- **WALBERG M ; GAUSTAD P ; STEEN HB.** Rapid assessment of ceftazidime, ciprofloxacin, and gentamicin susceptibility in exponentially-growing E-coli cells by means of flow cytometry. *Cytometry,* 1997, vol. 27, 169-178
- **WALLDEN M ; FANGE D ; LUNDIUS EG ; BALTEKIN Ö ; ELF J.** The synchronization of replication and division cycles in individual E. coli cells. *Cell,* 2016, vol. 166, 729-739
- **WANG J ; ZHANG Y ; XU D ; SHAO W ; LU Y.** Evaluation of the Sysmex UF-1000i for the diagnosis of urinary tract infection. *Am J Clin Pathol,* 2010, vol. 133, 577-582
- **WILLIS L ; HUANG KC.** Sizing up the bacterial cell cycle. *Nat Rev Microbiol,* 2017, vol. 15, 606-620
- **WILSON ML ; GAIDO L.** Laboratory diagnosis of urinary tract infections in adult patients. *Clin Infect Dis,* 2004, vol. 38, 1150-1158
- **WU JBY ; SHI CH ; CHU GCY ; XU QJ ; ZHANG Y ; LI QL ; YU JS ; ZHAU HYE ; CHUNG LWK.** Near-infrared fluorescence heptamethine carbocyanine dyes mediate imaging and targeted drug delivery for human brain tumor. *Biomaterials,* 2015, vol. 67, 1-10
- **YU H ; JING W ; IRIYA R ; YANG Y ; SYAL K ; MO M ; GRYS TE ; HAYDEL SE ; WANG S ; TAO N.** Phenotypic antimicrobial susceptibility testing with deep learning video microscopy. *Anal Chem,* 2018, vol. 90, 6314-6322
- **ZAMPIERI M ; SZAPPANOS B ; BUCHIERI MV ; TRAUNER A ; PIAZZA I ; PICOTTI P ; GAGNEUX S ; BORRELL S ; GICQUEL B ; LELIEVRE J.** High-throughput metabolomic analysis predicts mode of action of uncharacterized antimicrobial compounds. *Sci Transl Med,* 2018, vol. 10
- **ZHANG JH ; CHUNG TDY ; OLDENBURG KR.** A simple statistical parameter for use in evaluation and validation of high throughput screening assays. *J Biomol Screening,* 1999, vol. 4, 67-73
- **ZHENG H ; HO PY ; JIANG M ; TANG B ; LIU W ; LI D ; YU X ; KLECKNER NE ; AMIR A ; LIU C.** Interrogating the Escherichia coli cell cycle by cell dimension perturbations. *Proc Natl Acad Sci U S A,* 2016, vol. 113, 15000-15005